# EUROPEAN PATENT APPLICATION

(11) **EP 1 529 849 A2**
(43) Date of publication of application: **11.05.2005**
(21) Application number: 04447222.3
(22) Date of filing: 06.10.2004
(51) Int. Cl.: C12Q 1/68

(54) **Reliable method for assessing and reporting the risks of disease, contamination and losses caused by one or more microorganisms in a matrix or environment**

(30) Priority: 24.10.2003 US 693359 P
(71) Applicant: De Ceuster NV, 2860 sint-Katelijne-Waver (BE)
(72) Inventor: De Ceuster, Tom, 2860 Sint-Katelijne-Waver (BE); Lievens, Bart, 2860 Sint-Katelijne-Waver (BE); Vanachter; Alfons, 2860 Sint-Katelijne-Waver (BE); Krause, Matthew, 2860 Sint-Katelijne-Waver (BE); Thomma, Bart, Binnenhavens 5 6709 PD Wageningen (NL); Cammue, Bruno, Kasteelpark Arenberg 20 3001 Heverlee (BE)
(74) Representative: Brants, Johan Philippe Emile

(57) **Abstract**

The present invention relates to a method for assessing and reporting risks of disease, contamination or loss caused by one or more microorganisms in a matrix or an environment, said method comprising the steps of:
a) acquiring data regarding said one or more microorganisms and data regarding said matrix or said environment,
b) performing a risk analysis and assessment in order to determine a risk of disease, contamination or loss by said one or more microorganisms in said matrix or environment,
c) compiling the results of said risk analysis and outputting said results in a report, and
d) recommending suitable prevention, remediation or minimization strategies.
In a preferred embodiment, the invention comprises acquiring data regarding said microorganisms by detecting, identifying and quantifying one or more microorganisms or components thereof in said matrix or environment in a single assay, preferably using an array-based assay.

## Description

### Field of the invention

The present invention relates to a method and system for assessing and reporting the risks of disease, contamination and losses caused by one or more microorganisms in a matrix or environment. The present invention further provides a method and system for recommending a suitable prevention, remediation, or minimization strategy. The present invention further aims to provide a simple and fast diagnostic method and system enabling detection, identification and quantification of specific microorganisms from a possible presence of hundreds to thousands of distinct taxa in a matrix or environment.

### Background

The heavy reliance of professional agriculture and horticulture on a decreasing set of synthetic disinfectants and pesticides over the past century to manage plant diseases and environmental biological contaminants has resulted in severe side effects including negative impact on the environment, resurgence of the target diseases and development of resistance to individual pesticides in many target species.

Largely driven by environmental concerns, integrated pest management (IPM) is the disease management strategy that has been put on agricultural and horticultural production during the past few decades. IPM incorporates the use of different disease management strategies to reduce disease and pest incidence and severity to levels where minimal economic loss and environmental impacts occur. In contrast to the regular use of synthetic pesticide-based practices, IPM relies on strategies that limit or minimize the levels and frequency of synthetic pesticides use or may even eliminate their application (Apple and Smith, 1976, Integrated Pest Management Plenum Press, New York, USA). Preventive, pro-active pest management, which implies that action should be undertaken before actual disease occurs, is the "holy grail" of integrated pest management. However, preventive disease management and other forms of IPM are still lacking accurate diagnostics, risk assessment, and remediation recommendation tools, and their incorporation therein, to achieve optimum disease management and crop production with low environmental impacts.

The accurate and rapid detection and identification of potential pathogens is essential for effective disease control. Besides qualitative detection, quantitative measurement of pathogen biomass is one of the cornerstones of successful disease management enabling more informed decisions to be made about appropriate control strategies.

Until recently, pathogenic microorganisms were detected by culturing them on selective media or by biochemical, chemical and immunological analyses. These methods are often time consuming, laborious, or require extensive knowledge of classical taxonomy and are, as a consequence, subjective (McCartney et al. 2003 Pest Manag. Sci. 59:129-142). In addition, these methods may generate large numbers of false negative and false positive results. Furthermore, quantification based on conventional culturing methods is relatively inaccurate and unreliable (Goud and Termorshuizen 2003 Eur. J. Plant Pathol. 109:523-534; Jeffers and Martin 1986 Plant Dis. 70:1038-1043; Termorshuizen et al. 1998 Appl. Envir. Microbiol. 64:3846-3853; Thorn et al. 1996 Appl. Envir. Microbiol. 62:4288-4292; Tsao and Guy 1977 Phytopathology 67:796-801). Moreover, different microorganisms may disrupt or obstruct the detection of other microorganisms, even on selective media, thus complicating correct diagnosis.

Advances in molecular biology, and, in particular, the advent of polymerase chain reaction (PCR), have provided new tools for rapidly detecting and quantifying microorganisms without the need to culture. Additionally these new tools allow detection and quantification of non-culturable microorganisms, an aspect which is considerably important since possibly less than 1 % of microorganisms in an environmental sample can be cultured reliably (Amannet et al. 1995 Microbiol. Rev. 59:143-169). Using PCR, millions of copies of specific DNA sequences may be synthesized in a process referred to as amplification. Besides increasing the amount of a specific DNA fragment, this technique is routinely used as a diagnostic tool (Dieffenbach and Dveksler 1995 PCR Primer, A laboratory Manual, Cold Spring Harbor, USA, Cold Spring Harbor; Edel 1998 Polymerase chain reaction in mycology: an overview. In applications of PCR in Mycology, pp1-20 Eds Bridge, P.D., Arora, D.K., Reddy, C.A., Elander, R.P., Wallingford, UK, CAB International). If a DNA sequence is known to be unique to a particular organism, specific PCR primers can be designed to enable determination of the presence or absence of the organism in question in a sample of interest. For instance, US 5,527,671 describes the characterization of a DNA fragment that is characteristic of *Verticillium dahliae* and the use of PCR-based technology to detect this specific DNA fragment for diagnosing whether or not this fungus is present in a sample. Multiplex PCR assays may be used when simultaneous detection of several microorganisms is needed. Multiplex PCR assays employ several PCR primers in the same reaction, thus reducing analysis time and costs. However, the development of efficient and reliable multiplex PCR methods can be problematic and typically requires extensive strategic planning and optimization of reaction conditions in order to properly discriminate at least a few amplicons per reaction.

Real-time PCR has been a powerful development, especially with regard to quantification purposes (Heid et al. 1996 Genome Res. 6:986-994). This technology is more sensitive, more accurate and less time consuming than conventional, end-point quantitative PCR because it monitors PCR products as they accumulate during the reaction. This allows template quantification during the exponential phase of the reaction, before reaction components become limiting. Increasingly, real-time PCR is being used for accurate detection and quantification of specific plant pathogens as well as for monitoring pathogen infection (Brouwer et al. 2003 FEMS Microbiol Lett. 228:241-248; Schaad and Frederick 2002 Can. J. Plant Pathol. 24:250-258). Although not yet applied to plant pathogens, using multiplex formats enables simultaneous detection and quantification of different organisms (Boivin et al. 2004 J. Clin. Microbiol. 42:45-51; Candotti et al. 2004 J. Virol. Methods 118:39-47). However, the total amount of PCR reactions in a single tube is severely limited by the number of different fluorescent dyes available and the common use of a monochromatic energizing light source in real-time PCR instruments (Mackay et al. 2002 Nucleic Acids Res. 30:1292-1305). As a result, detection of more than a handful of different pathogens is currently difficult and unreliable whether with conventional or real-time PCR

In many applications, especially in plant disease diagnostics, potential pathogenic organisms present in biological samples are numerous, abundant and belong to different (sub)classes, orders, families, genera, species, subspecies, and even individuals. As a consequence, multiplex qualitative and quantitative detection, enabling to detect and identify a large number of specific organisms, is a major challenge in plant disease diagnostics and plant disease management, especially since the approaches mentioned above are inconvenient and costly when more than a few organisms need to be detected simultaneously.

In contrast, array technology can be used to detect an in principle unlimited number of different organisms in a single assay (Lévesque 2001 Can. J. Plant Pathol.24:333-336, Martin et al. 2000 Annu. Rev. Phytopathol. 38:207-239). The advantage of this technology is that it combines nucleic acid amplification with the unlimited screening capability of arrays, resulting in high degrees of sensitivity, specificity, and throughput capacity. With this technology, detector probes, also known as capture probes, are spotted on a solid support to create the array. Then, in general, the target DNA is amplified with conserved PCR primers (for RNA-based targets after a reverse transcription step), labeled, and subsequently hybridized to the array under stringent conditions. This technology was originally developed as a technique to screen for human genetic disorders (Saiki et al. 1989 Proc. Natl. Acad. Sci. USA 86: 6230-6234), but has also been successfully applied to detect and identify human and animal pathogens (Anthony et al. 2000 J. Clin. Microbiol. 38:781-788; Fiss et al. 1992 J. Clin. Microbiol. 30:1220-1224; González et al. 2004 J. Clin. Microbiol. 42:1414-1419). In plant pathology, DNA array technology has been successfully applied to identify oomycete, nematode, and bacterial pathogen DNA from pure cultures (Fessehaie et al. 2003. Phytopathology 93:262-269; Lévesque et al. 1998 Phytopathology 88:213-222; Uehara et al. 1999). Recently, the utility of DNA arrays for multiplex detection and identification of plant pathogens from complex environmental samples, including those derived from soils and plants, was demonstrated (Lievens et al. 2003a Commun. Agric. Appl. Biol. Sci. 68:569-81, Lievens et al. 2003b FEMS Microbiol. Lett. 223:113-122). An expanded version of the latter test is nowadays commercially available for detection and identification of microorganisms in biological samples. A major limitation of the current format, however, is that reliable quantification of pathogen presence is not yet available. Hence, only qualitative detection has been possible.

US patent application No 2003/0198943 describes an identification and/or quantification method of a large number of organisms using array technology. The reported method is based on the use of arrays to discriminate between homologous genetic sequences belonging to several groups of organisms together with the identification of these groups as such. This technique is described to be particularly suitable for identifying, genotyping and classifying organisms based on differences in DNA or RNA sequences, for identifying the origins of food products such as meat, fish, plants, or for identifying genetically modified organisms (GMO).

A major challenge for accurate plant disease management strategies is to provide a correct and reliable prediction system, able to assess and reveal the risks that pathogenic or other organisms present in a matrix or environment will cause disease, contamination, and economical loss. However, a great need remains for such prediction system, especially since suitable methods for detecting and quantifying microorganisms are currently lacking. In conclusion, a centralized formal framework for diagnosis, risk assessment, and reporting is necessary.

The present invention aims to provide a reliable method for assessing and reporting the risks of disease, contamination and losses caused by one or more microorganisms in a matrix or environment

The present invention further aims to provide a simple and fast diagnostic method enabling the detection, identification and quantification of specific microorganisms from a possible presence of hundreds to thousands of distinct taxa in a matrix or environment.

### Summary

A major challenge for plant disease management strategies is to provide a correct and reliable advice, even before the actual disease occurs. Therefore, there is a great need in the art for a prediction system, able to predict the risk that pathogenic microorganisms that are found to be present in a matrix or environment will cause disease, contamination or economic losses. However, especially since suitable methods for detecting and quantifying multiple pathogenic and beneficial organisms and any kind of disease and contamination risk assessment systems are still lacking, decisions to be made about controlling potential pathogens and contaminants are not confirmed or justified.

In a first aspect, the present invention relates to a method for assessing and reporting risks of disease development, contamination, or losses, caused by one or more microorganisms in a matrix or environment, preferably associated with agriculture or horticulture. More in particular, the present invention provides a method for assessing and reporting a risk of disease development, contamination, or loss by one or more of the detected, identified and quantified microorganisms and for reporting said risk. In a preferred embodiment, the present method comprises the steps of:
a) acquiring data regarding said one or more microorganisms and data regarding said matrix or said environment,
b) performing a risk analysis and assessment in order to determine a risk of disease, contamination or loss by said one or more microorganisms in said matrix or environment, and
c) compiling the results of said risk analysis and outputting said results in a simple, straightforward and understandable report.

In a particularly preferred embodiment, the invention comprises a method whereby data regarding said microorganisms is at least partly acquired by detecting, identifying and quantifying one or more microorganisms or components thereof in said matrix or environment in a single assay, preferably an array-based assay. In another preferred embodiment, the present method further comprises the step of recommending an appropriate disease, contamination, and loss prevention, remediation or minimization strategy.

The present invention provides a method which is at least partly based on accurate and reliable detection, identification and quantification of pathogenic, non-pathogenic, and contaminant microorganisms. The present method, as claimed in present claim 1, permits to distinguish specific detrimental as well as beneficial microorganisms from the likely presence of hundreds to thousands of distinct taxa in the tested sample (step a). The method further enables to estimate the risk of development and outcome of diseases in this matrix or environment (step b). For that, the present method performs a risk analysis wherein not only the presence, identity and quantity of microorganisms as defined in step a) are taken into account but wherein also specific matrix and environmental characteristics are considered. In addition, the present method provides in step c) a simple and easily interpreted risk analysis report and recommends a suitable preventive, preemptive, corrective, curative or terminative action that conforms to the specific matrix or environmental characteristics and to the actual risk of disease, contamination and loss. The present invention is composed of three essential interdependent elements: 1) client-level data acquisition, 2) intelligent disease risk assessment, and 3) results and recommendation reporting. The invention provides a concept that forms the basis for environmentally sound plant health management. The invention is intended to be fully integrated into decision making required for preventative, preemptive, curative, and terminative plant health, environmental quality, and microbial contamination management. It will enable more informed, justified decisions to be made for controlling diseases about, for instance, plant species or cultivar choice and how and when chemicals can be used most effectively to control disease epidemics.

In a second aspect, the present invention provides a complete method for identification, detection and quantification of plant-pathogenic, contaminant as well as non-pathogenic microorganisms in a matrix or environment, preferably those associated with agriculture and/or horticulture. In a particularly preferred embodiment, said method comprises the steps of detecting, identifying and quantifying one or more microorganisms or components thereof using an array-based assay, preferably a multiplex array-based assay, I.e. a assay wherein multiple microorganisms may be detected, identified and quantified.

This method for identification, detection and quantification of microorganisms in a matrix or environment may further be linked to a method for assessing and reporting a risk of disease development, contamination, or loss caused by said detected, identified and quantified microorganisms.

Those experienced in diagnostics and addressing disease or contamination threats will immediately recognize the many other strengths and advantages of the present invention and the numerous possibilities for end uses of the present invention from the detailed description and examples provided below.

### Detailed description of the figures

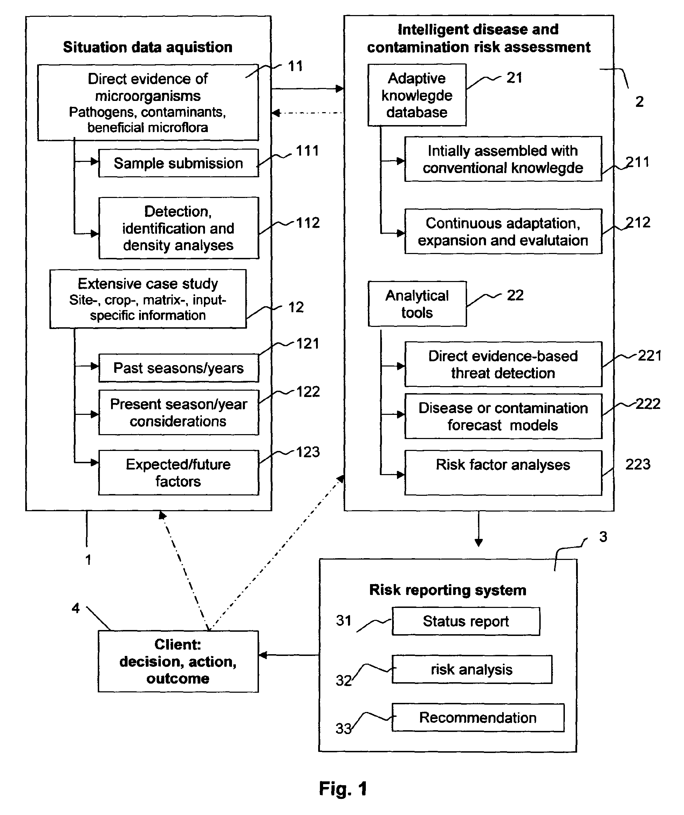

**FIG. 1** is a schematic representation of the method and system according to the present invention for assessing and reporting a risk of disease development, contamination, and loss caused by one or more microorganisms in a matrix or environment.

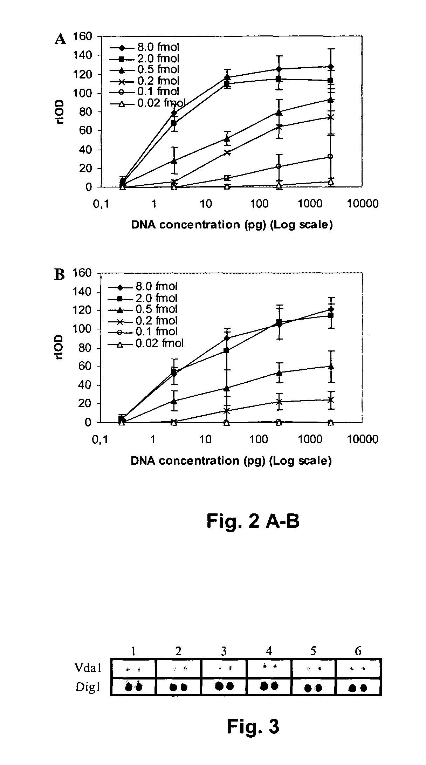

**FIG. 2** illustrates the quantification of a dilution series of *Verticillium dahliae* (A) and *V. albo-atrum* (B) genomic DNA using different amounts of detector probes on a DNA array. Genomic DNA was preamplified and simultaneously labeled. Detector probes were spotted on a pre-activated nylon membrane at several amounts ranging from 0.02 to 8.0 fmol. Hybridization signal strength is reported relative to the average integrated optical density of a labeled reference control, namely a digoxigenin-labeled control- (rIOD), and plotted versus the logarithmic DNA concentration. Data represent the average of 2 independent analyses of hybridization signals (n = 4) using detector sequences Vda1 and Val2, to detect *V. dahliae* and *V. albo-atrum* respectively. The error bars indicate standard deviations. The experiment was repeated twice with similar results.
**FIG. 3** shows the influence of non-target DNA on hybridization signals. Signals after hybridization of labeled amplicons resulting from co-amplification of 0.25 pg *V. dahliae* and 250 pg non-target DNA to the oligonucleotide detectors Vda1, to detect *V. dahliae,* and Dig1, a digoxigenin-labeled reference control for calibration. Detectors were spotted in duplicate at 8.0 fmol on a pre-activated nylon membrane. DNA isolated from a bacterial (*Rhizobium vitis*; 1), oomycete (*Pythium ultimum*; 2), or fungal (*Fusarium solani*; 3) culture or from tomato plant (4) or sandy soil (5) were used to test possible interference. In panel 6, no non-target DNA was added.

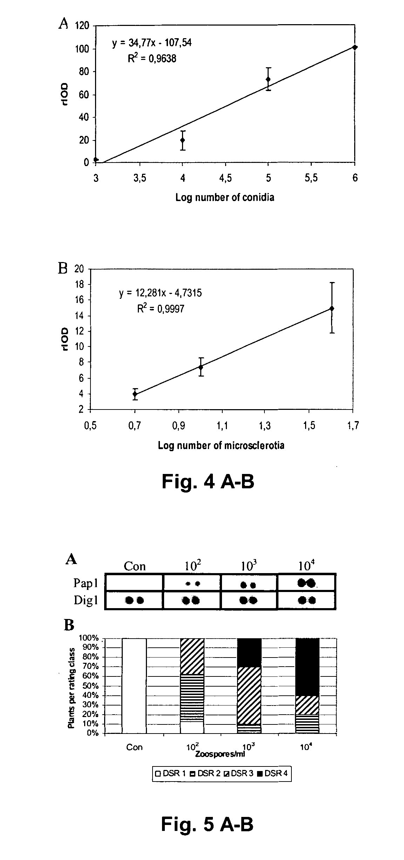

**FIG. 4** shows a quantitative assessment of *V. dahliae* presence in artificially infested soil samples after PCR preamplifcation. Detector probes were spotted at 8.0 fmol on a pre-activated nylon membrane. (A) Regression line for DNA array analysis of a 10-fold dilution series of *V. dahliae* conidia added to 0.75 g soil. (B) Regression line for DNA array analysis of a series of 40, 10, and 5 microsclerotia from *V. dahliae* added to 0.75 g soil. Hybridization signal strength is reported relative to the average integrated optical density of a labeled reference control, namely a digoxigenin-labeled control (rIOD) and plotted versus the logarithmic number of pathogen propagules. Data represent averages of hybridization signals generated by Vda1, a detector probe to specifically detect *V. dahliae,* from 2 independent analyses (n = 4). Error bars indicate standard deviations.
**FIG. 5** illustrates a quantitative assessment of *Pythium aphanidermatum* in artificially infested water-based samples. (A) DNA array analysis 10 days after inoculation of nutrient solution with 0 (Con), 10², 10³ or 10⁴ *P. aphanidermatum* zoospores/ml. Signals after hybridization of labeled amplicons to the oligonucleotide detector probes to detect *P. aphanidermatum* (Pap1) and the digoxigenin-labeled reference (Dig1) are shown. Detector probes were spotted in duplicate at 8.0 fmol on a pre-activated nylon membrane. (B) Disease severity rating (DSR) for root and foot rot expressed as the percentage of plants per treatment (n = 10) is presented. Plants were rated 10 days after treatment for disease severity on a 1 to 5 scale: 1=symptomless, 2=light browning and/or superficial lesions present, 3=dark browning and/or sunken lesions present, 4=development of coalescing lesions and necrosis, 5=plant death. The experiment was repeated twice with similar results.

### Detailed description of the invention

In order to determine the best strategy for effective plant disease prevention and/or remediation in a given matrix or environment, it is important not only to know which microorganisms -whether pathogenic, non pathogenic or contaminant- are present but also to what degree. In plant pathology, the imposed strategy of disease management is not simply to combat a pathogen whether or not it is present, but to apply corrective measures only when its presence is confirmed and its magnitude is expected to result in disease development, increasing distribution and inoculum potential and/or economic loss.

### Method for assessing and reporting risks

In a first embodiment the present invention provides a method for assessing and reporting risks for disease development, contamination, and losses caused by one or more microorganisms in a matrix or environment comprising the steps of:
- acquiring data regarding said one or more microorganisms and data regarding said matrix or said environment,
- performing a risk analysis and assessment in order to determine a risk of disease, contamination or loss by said one or more microorganisms in said matrix or environment,
- compiling the results of said risk analysis and outputting said results in a report, and
- recommending an appropriate prevention, remediation or minimization strategy.
The present method is essentially composed of three interdependent steps: 1) a data acquisition step whereby data on the environment, and the microorganisms present herein is acquired, 2) a risk assessment step wherein the risk of pathogen disease development is analyzed and 3) a reporting and advising step wherein the results of the risk analysis are presented and wherein disease prevention, treatment or minimization strategies are reported. The present invention thus provides for a method which is particularly suited for the detection, identification and quantification of microorganisms, in particular plant-pathogenic and non-plant pathogenic organisms and contaminants. The present method also permits to determine a risk of disease development, contamination or loss in said matrix or environment, to asses this risk, to report the results of risk analysis and assessment in a simplified report and to recommend suitable and adapted strategies for appropriate disease prevention or remediation or minimization treatments. Such recommendation may include but are not limited to the recommendation of actions to prevent, preempt, remediate, and minimize pathogen, contaminant, or disease development, or associated losses.

As used herein, the term "*detection*" refers to determining the presence of a microorganism or its components. The term "*identification*" means discernment of a detected microorganism according to a specific taxonomic definition. The level of taxonomic identity is dependent on a) the best available level of differentiation possible by a given identification method, approach or system, b) the most current classification information available, and c) the degree of taxonomic specificity appropriate to pathogen, host, environment, matrix, or other sample source specialization, or particular situation. The terms "*quantification*", "*density analysis*" and *"density determination"* are used interchangeably to refer to the relative determination of the extent or magnitude of microbial presence (e.g. colonization, contamination, inoculum potential, etc.) in a given matrix or environment on a given scale. It shall be understood that this term indicates the determination of an appropriate quantity which is sufficient for carrying out a meaningful and reliable risk analysis and assessment thereon, in accordance with the present invention.

In the context of the invention, "*matrix*" refers to any substance occupying a sampling area or volume, any object or medium from which a sample is drawn, or a physical sample itself. Examples of *matrix* include but are not limited to soil, water, air, plant tissue and residues, seeds, potting media, composts, stonewool, pure fungal bodies, pure bacterial biomass, filtrates, plant extracts, nutrient solutions, and collected surface washings. *"Environmenf"* comprises a system, setting, habitat, niche, domain, atmosphere or other location designation surrounding the object or sample being evaluated. This term implies but is not limited to the spatial context and scale relevant to the object, object-related data, or specific activity under study. Several samples from one or more matrices may be collected and analyzed from an environment.

In accordance with the present invention, a sample collected from a matrix and an environment, as defined above, is intended to be analyzed for detection, identification and density determination of microorganisms in the matrix and environment containing the matrix. It should be stated that any type of sample composed of any type of matrix could be collected from any environment. Samples from matrices may include but are not limited to air, soils, other growing matrices (e.g., potting media, stonewool, etc.), plant material (e.g., seeds, transplants, produce, raw products, processed plant parts, plant residues, etc.), and other components and inputs (e.g., irrigation system components; nutrient solutions; ground and surface water supplies; substrate and soil amendments; organic and probiotic nutrients; handling, processing and transport elements; plowing, planting, cultivating, and harvesting implements; etc.). In addition, samples may also be derived from steps, products or components of other diagnostic methods such as but not limited to: fungal and bacterial colonies or biomass cultured on non-selective, enrichment, semi-selective and selective media; certain amplicons generated for different RNA- or DNA-based methods; propagules collected from air, liquid, and various surfaces, and classified and enumerated by microscopic examination; etc.).

In this invention, "*microorganisms*" refers to microscopic organisms including but not limited to fungi, actinomycetes, bacteria, mollicutes including mycoplasma and phytoplasma, protozoa, stramenopiles, nematodes, viruses, viroids, and prions (infectious protein particles). Living organisms including microorganisms are systematically described, named, and classified based on their presumed relationships to one another. Classification is based on a hierarchical placement of organisms within different levels of specificity or ranks in the taxonomic system. The most universal taxonomic system uses the ranks kingdom, phylum, class, order, genus, and species, whereby kingdom and species represent the largest and smallest classification units, respectively. Beyond species, other ranks can be applied for increasing levels of specificity such as subspecies, biotype, race, variety, strain, and isolate. As used herein, "*group of microorganisms*" refers to microorganisms that share a particular character or set of attributes and can be applied at any level of the classification hierarchy.

In the preferred context of this invention, said microorganisms shall include plant-pathogenic, non-plant pathogenic, and contaminating microorganisms. "*Plant-pathogenic microorganism*" refers to a microorganism that has the capacity to cause disease in a particular susceptible plant host or range of plant hosts. Conversely, *"non-plant pathogenic microorganism"* refers to a microorganism that is *not* able to cause disease on a particular plant host or range of hosts, but may include "*beneficial microorganisms*", which are those microorganisms that either directly or indirectly substantially promote or improve the well-being of a particular plant host, range of plant hosts, and/or plant environment. *"Contaminating microorganism"* and "*microbial contaminant*" both interchangeably refer to a microorganism able to infest, increase its population in, and degrade the quality (physical, chemical, biological, regulatory, or marketable) of a matrix or environment, or whose presence and reproduction in such a matrix or environment serves as an intermediate for promoting disease in another matrix or environment. All of these classifications of microorganisms include but are not limited to fungi, actinomycetes, bacteria, mollicutes, protozoa, stramenopiles, nematodes, viruses, viroids, and prions,

### Data acquisition

In a first step, the present method provides for the acquisition of data regarding one or more microorganisms and data regarding said matrix or said environment. Advantageously, the detection, identification and quantification of one or more microorganisms or components thereof can be performed in a single assay, which is time and cost effective. Moreover, using a single assay it is possible to test for an unlimited number of different organisms.

Said detection, identification and quantification method is not considered to be limitative and may comprise any technique for detecting, identifying and quantifying microorganisms. In a preferred embodiment said method comprises RNA or DNA-based analysis method, or protein and immunological assays, or classical culture-dependent and microscopy-based methods. More preferably said one or more biological organisms or components thereof are detected, identified and quantified in a single assay using an array-based system.

As used herein the term "*data regarding organisms*" does not only refer to data regarding the detection, identification and quantification of one or more biological organisms but also refers to additional data including but not limited to taxonomical classification of the organisms; amount; type e.g. soil-born; infectivity; toxicity; pathogenicity (i.e. property or an organism of causing disease); virulence (i.e. the degree of an organism to cause a disease e.g. avirulent, weakly or highly virulent); production of non-cellular entities such as toxins and allergens; nutrient and environmental requirements; reproductive cycle e.g. biotrophic, hemi-biotrophic; necrotrophic, aerobic, anaerobic; infection mode; replication rate; host range; presence of genes in the organisms which confers resistance; history of prior genetic modifications to the organisms; stability of the genetic traits of the organisms; vectors of disease transmission of the organisms; base sequence, frequency of mobilization and specificity of the organisms' indigenous vectors; the organisms' other potentially significant physiological traits and the stability of those traits; organisms' natural habitat and geographical distribution; climatic characteristics of the organisms' natural habitat; involvement of the organisms in environmental processes including nitrogen fixation and pH regulation; interaction of the organism with other organisms in the environment and its effect on those organisms including its likely predation, parasitic, competitive or symbiotic properties; ability of the organism to form survival structures including seeds, spores or sclerotia; colonization and propagation rate; survival; establishment in the environment; etc...

The present method also comprises the step of retrieving data regarding the diagnosed environment. As used herein the term *"data regarding the matrix or the environment*" refers to data including but not limited to geographical location of the environment; climate conditions e.g. weather conditions, humidity, temperature, season; soil type or soil substrate type e.g. sand, loam, clay; stonewool, potted soil; soil conditions e.g. aeration level, temperature, (ground) water level, humidity level, degree of compaction; (crop) plant conditions e.g. growing and harvesting season, growth cycle, stage of development; crop variety, chemical, physical and nutritional requirements; environmental requirements, resistance level; sensibility to pathogens; degree of natural or acquired immunity against pathogens; culture conditions such previous and/or subsequent crops in a crop rotation system, crop production methods, e.g. crops grown in the open field, in a growth chamber or in a greenhouse; crop yield; land/facilities use and modification; adjacent plants and land features, site topography, sources, composition and other input attributes, disease and pest events, executed and planned preventive and/or curative disease management strategies; factors affecting survival, multiplication and dissemination of organisms in the environment; ecosystems; physical and chemical parameters; government regulations and policy, market value and foreseen production costs, etc...

### Risk analysis step

A further step in the method according to the present invention consists of performing a risk analysis and assessment in order to determine a risk of disease development, contamination or loss by said one or more microorganisms in said matrix or environment.
In the context of the invention, "*risk*" refers to the probability of incurring substantial disease, contamination, and/or economic or material loss will occur as a result of exposure to, presence of, and/or favorable conditions for development of pathogenic or contaminating microorganisms. "*Threaf*" in the context of the proposed invention refers to an impending physical and economic loss due to disease, contamination, or similar injury or compromise in plant, crop, or product quality and value. The term "*loss*" in the context of the invention is the diminution or destruction of quality, function, presence, or value due to disease, contamination, disorder, or other injury to a plant, crop, plant-derived product, matrix, or environment. Loss may be economic, aesthetic, or epidemiological in nature. "*Disease*" refers to: 1) any disturbance of a plant, crop, plant-derived product, or environment that interferes with its normal growth or progress, function and development, economic value, or aesthetic quality, and leads to development of symptoms; 2) a continuously, often progressively affected condition in which any part of a plant, crop, plant derived-product, matrix, or environment is abnormal or that interferes with the normal activity, appearance, quality or function of its components (e.g., cells, organs, structural features, chemical/biochemical properties, etc.). *"Contamination"* refers to the rendering of an otherwise pure substrate, matrix, environment, or system unclean or unsuitable for its intended purpose by a microorganism, its growth and activity, or byproduct produced therein.

In a particularly preferred embodiment of the present method a risk analysis is performed by using a risk analysis and assessment system comprising an adaptive knowledge database, preferably comprising data on biological organisms, crops, diseases and environmental, and analytical tools preferably related to: A) direct evidence-based threat detection, B) disease or contamination forecast models and C) risk factor analyses.

### Reporting step

In a further embodiment, the present invention provides a method comprising compiling the results of the risk analysis and outputting said results in a simple report. The report may include any type of report such as but not limited to a written or narrative report, available by mail, email, fax, telephone, etc...

In a particularly preferred embodiment, the report indicates risk levels for all possible disease development, contamination or loss. These risk levels are preferably indicated by means of visual codes. According to a preferred embodiment of the invention, a method is provided wherein the report comprises visual codes wherein each code corresponds to a category of risk of disease development, contamination or loss. Non-limitative examples of suitable visual codes include for instance graphs, charts, symbols, letters, numbers, drawings, colors, words, signs such as a plus or minus sign, a visual display, an image, a scheme, a photograph, etc... Color codes are particularly preferred in accordance with the present invention.

In accordance with the present invention, a report is provided wherein each code corresponds to a category of risk of disease development, contamination or loss. Possible risk categories may include but are not limited to no risk, negligible risk, low risk, moderate risk, high risk categories. In a preferred embodiment, a report obtained in accordance with the present method comprises at least three different codes corresponding to three categories of risk of diseases development, contamination or loss comprising: a first category indicating a no risk of diseases development, contamination or loss, a second category indicating a low risk of diseases development, contamination or loss, and a third category indicating a high risk of diseases development, contamination or loss. More preferably, a report obtained in accordance with the present method comprises at least five different codes corresponding to five categories of risk of diseases development, contamination or loss including categories indicating a no risk, negligible risk, low risk, moderate risk, and high risk of damage and/or disease development. Even more preferred, said five categories correspond to five color codes.

### Recommendation step

In accordance with the present invention, the present method further comprises the step of recommending an appropriate prevention, remediation or minimization strategy for each category of risk of damage and/or disease development. For example, in a preferred embodiment, the method determines that for a "no risk" category prevention, remediation or minimization are not required. In another preferred embodiment the method determines that for a "low risk" category a suitable monitoring of the condition is required. In yet another preferred embodiment, the method determines that an appropriate prevention, remediation or minimization strategy is required for a "high risk" category. Therefore, in a preferred embodiment, the above mentioned report further comprises recommendation advice regarding a suitable prevention, remediation or minimization strategy, and for instance a representation of all possible options to prevent, preempt, correct, or terminate existing or anticipated each epidemic or contamination.

In a preferred embodiment, the present invention also relates to a risk analysis report obtainable by performing a method according to the present invention, wherein said report comprises visual codes whereby each code corresponds to a category of risk of damage and/or disease development and optionally also to a suitable prevention, remediation or minimization strategy.

### System for assessing and reporting risks

In yet another embodiment, the present invention relates to a system for assessing and reporting a risk of disease development, contamination or loss, by one or more microorganisms in an environment. In particular, this system comprises:
- data acquisition means for acquiring data on one or more microorganisms and on said matrix or environment:

- a risk analysis and assessment system for analyzing and assessing the risk of disease development, contamination or loss, by said one or more microorganisms, and
- a risk reporting system for outputting the results of said risk analysis and assessment in a suitable report.

A preferred embodiment of a system according to the present invention is schematically illustrated in **Fig. 1**, and will be discussed into more detail hereunder in example 1.

In addition, in another preferred embodiment, the present system further comprises a computer system adapted for performing a method according to the present invention. Said system preferably comprises: a processor; a memory comprising software instructions adapted to enable the computer system to perform tasks, wherein the software instructions comprise but are not limited to inputting data regarding microorganisms or the matrix or environment, retrieving data regarding microorganisms or the matrix of the environment; adjusting a plurality of risk analysis parameters for application to the inputted and retrieved data; analyzing the inputted and retrieved data, outputting a risk analysis in a report, etc...

### Method for detecting, identifying, and quantifying microorganisms

In another aspect, the present invention provides a method for the detection, identification and quantification of one or more microorganisms or components thereof in said matrix or environment, preferably an agricultural and/or horticultural environment. The invention is especially suited for the detection, identification and quantification of specific plant-pathogenic, beneficial microorganisms and contaminants from a possible presence of hundreds to thousands of distinct organisms.

In a preferred embodiment of the invention one or more microorganisms are detected, identified and quantified according to the subsequent steps of:
- extracting targets from a matrix or environment,
- contacting said targets, preferably after an amplification step, with capture (or detector) molecules bound to a solid support; and
- detecting and quantifying signals resulting from the specific binding between said targets and their corresponding specific capture molecules,
wherein said capture molecules are bound to a solid support at a specific location in an array format,
wherein the binding between targets and their corresponding capture molecules forms said signals at the expected location and
wherein said signals are correlated to the detection, identification and quantification of specific microorganisms or components thereof.

Preferably, in accordance with said method one or more microorganisms or components thereof are detected, identified and quantified in a single assay using an array-based system.

The term "*array*" according to the invention refers to the format in which capture molecules, for instance taxon-specific nucleic acids, have been arranged at defined locations on a solid support, either by spotting or direct synthesis. The type of array used in the present invention is not to be considered as limitative and may comprise any kind of arrays, including DNA arrays, RNA arrays, protein arrays, macroarrays, microarrays and chips. Whereas with microarray technology several capture molecules can be spotted on little more than a square mm, the number of spots is relatively less for macroarrays. On the other hand macroarrays typically have bigger spots, thus containing more capture molecules.

The term "*solid support*" as used in the present invention refers to the support to create the array and can be made with materials selected from, but not limited to, the group consisting of nylon, (nitro)cellullose, glass, electronic devices, silicon supports, plastic supports, silica, metal or a mixture in format such as membranes, slides, compact discs, gel layers, microbeads. Preferably the solid support upon which capture molecules are arrayed comprise glass slides or nylon membranes.

The term *"capture molecules"* (also known as detector molecules) refers to molecules, e.g. taxon-specific nucleic acids, that are attached to the said solid support to "capture" the corresponding target molecules.

The term *"targets"* as used herein refers to molecules extracted from a sample. The term "molecules" should be understood in its largest sense, including but not limited to nucleic acids, proteins, metabolites, primary or secondary metabolites, enzymes, receptors, antibodies, etc... In a preferred embodiment, said target molecules comprise genomic DNA, genomic RNA or mRNA (messenger RNA). To increase sensitivity, preferably said targets are amplified. In the case of RNA targets, RNA may be converted into cDNA (copy DNA) by a reverse transcription step. After labeling, targets are hybridized to an array of specific capture molecules affixed to a solid support.

The term *"components thereof"* as used herein refers to any nucleotide sequence or amino acid sequence (peptide) specific to or derived from said microorganism and to other components of microorganisms such as proteins, primary or secondary metabolites, enzymes, receptors, antibodies, etc...

Advantageously, the present method comprises detecting, identifying and quantifying the presence of several specific microorganisms or components thereof, from a possible presence of hundreds to thousands of distinct taxa in a matrix or environment by correlating the signals obtained from the specific binding between the targets and their corresponding specific capture molecules to the presence, identity and density of said microorganisms or components thereof in said sample and thus said environment.

A first step in the present method for detecting, identifying and quantifying microorganisms or components thereof in a matrix or environment comprises taking an appropriate, representative sample of said matrix or environment. It should be clear that any type of sample can be taken from this environment.

A next step comprises the extraction of targets, as defined herein and preferably genomic DNA, genomic RNA or mRNA from said sample. Extraction techniques are well known in the art and are therefore not further discussed.

To increase sensitivity, a subsequent step in the present method may comprise target amplification. The amplification process may, for instance, employ double or single stranded DNA, cDNA or RNA. Also proteins or other target molecules may be amplified.

The amplification step used in the present invention is advantageously obtained by well known amplification protocols, preferably selected from, but not limited to the group consisting of polymerase chain reaction (PCR), reverse transcription-PCR (RT-PCR), ligase chain reaction (LCR), nucleic acid sequence based amplification (NASBA), transcription mediated amplification (TMA), and rolling circle amplification (RCA). According to the invention, a preferred method for target amplification is PCR, preferably pursued by using consensus PCR primers.

According to the invention, the method further comprises the amplification of exogenous and/or endogenous control molecules. For instance, exogenous and/or endogenous DNA can be (co-)amplified with said extracted DNA or cDNA during DNA amplification. Preferably, amplification is carried out using:
- at least one consensus primer pair for amplifying target molecules, preferably DNA, from a specific group of microorganisms, and
- at least one primer pair for amplifying exogenous and/or endogenous control molecules, preferably control DNA.

Said amplifications can be carried out in separate reactions, but preferably in a single amplification reaction, e.g. a PCR reaction.

Said primer pair specific to a group of microorganisms preferably comprise two anti-parallel consensus primers which hybridize to a sequence shared by all or most members of said group of microorganisms. Said primer pair specific to a group of microorganisms may thus comprise a primer pair specific to for instance but not limited to fungi, oomycetes, bacteria, viruses, viroids, nematodes, mycoplasma, or phytoplasma. It should be understood that said primer may also comprise a primer pair which is specific to a subdivision of such groups (e.g. a primer pair specific to certain branches, classes, subclasses, orders, families, genera, species, subspecies or varieties within a group) such as e.g. ascomycetes and basidiomycetes, both belonging to the group of fungi. An example of a primer pair for amplifying ribosomal DNA from most fungi is ITS1-F and ITS4 (Gardes and Bruns 1993). OOMUP18Sc and ITS4 may be used to specifically amplify ribosomal DNA from most oomycetes (Lievens et al. 2004 Plant Dis. 88:86) (see example 2).

Exogenous and/or endogenous control molecules may comprise endogenous and/or exogenous nucleotide sequences. Preferably, endogenous and/or exogenous DNA is used. Exogenous DNA is added to the amplification reaction at a specific and fixed amount, preferably comparable with the amount of target DNA expected in a sample. Exogenous DNA may comprise any DNA that is not expected to be naturally present in the tested sample. Endogenous DNA may comprise any DNA that is naturally present in the tested sample. Such endogenous DNA may for instance comprise plant-derived DNA in the case a plant sample has to be analyzed. Exogenous DNA may for instance comprise DNA derived from baker's yeast (*Saccharomyces cerevisiae*), which is exogenously added to the DNA sample to be tested. A primer pair for amplifying said endogenous and/or exogenous DNA comprises a primer pair, which is specific for said endogenous and/or exogenous DNA. In an example, said exogenous DNA derived from *S. cerevisiae* may be amplified by using the *S. cerevisiae*-specific primer pair P450₁ and P450₂ (Morace et al. 1997 J. Clin. Microbiol. 35:667-672). In another example, said endogenous DNA may comprise DNA from the plant *actin* gene, which is endogenously present in a plant-derived sample.

The exogenous control can be added in the initial biological sample, but preferably after the extraction step (DNA). Preferably, the hybridization yields of the control (exogenous or endogenous) is identical or differ no more than 30%, preferably no more than 20% and more preferably no more than 10% from the hybridization yield of the targets.

It should be clear from the present invention that more than one primer pair for amplifying endogenous and/or exogenous control molecules, such as control DNA can be used. It should also be clear from the present invention that more than one group-specific primer pair for amplifying target DNA from the target groups of microorganisms may be used. If one pair of consensus primers is not enough to amplify all the desired homologous sequences, then two or more primer sets may be used in order to obtain the desired amplifications. The minimum homologous sequences amplified by the same consensus primer pair is two.

Advantageously, target DNA consists of variable sequences, specific to the target organism, and flanked conserved sequences which can be used as consensus primer targets. The length of said target DNA is selected to be preferably between 20 and 1500 bases, and preferably between 50 and 1000 bases. Too long target fragments may reallocate faster and adopt secondary structures, which can inhibit hybridization to the corresponding capture molecules. However, the amplified target nucleotide sequence can be cut before hybridization.

According to the invention, the capture nucleotide sequence is a sequence having between 15 and 500 bases, preferably between 18 and 300 bases. In order to obtain an effective bound to the solid support and to avoid steric hindrance, this sequence is linked to a spacer. Said spacer is a chemical chain of at least 6.8 nm long (or at least 4 carbon groups), a nucleotide sequence of more than 15 bases or a nucleotide derivative.

For sensitive detection using arrays, target pre-amplification is required. Although other amplification techniques might be used, PCR amplification is preferred according to the present invention. There are, however, limitations to the use of PCR in a quantitative end-point approach, since bias in template-to-product ratios may be introduced due to typical PCR amplification kinetics. To ensure amplicon detection at the exponential (semi-linear) phase of the PCR reaction, according to a particularly preferred embodiment the method according to the invention comprises adjusting the number of amplification cycles to a number of between 25 and 40 cycles, and preferably to a number of between 25 and 35 cycles. In theory PCR response is exponential, but after a certain number of cycles a plateau level is reached e.g. due to the shortage of reagents, a reduced efficiency of DNA polymerase by the many heating and cooling cycles, etc. In order to be effective for end-point quantification, a hybridization reaction should be carried out with amplicons from this exponential phase. It was demonstrated that performing between 25 and 35 PCR cycles results in a sensitive detection and accurate quantification, since under these conditions, PCR response still occurs in the exponential stage.

Said amplification step -if pursued- is followed by a step to selectively detect, identify and quantify the target organisms, including pathogenic, non-pathogenic and contaminant microorganisms. Said detection and identification is advantageously obtained by hybridization of target fragments to capture molecules arranged at specific locations in an array and bound to a solid support. The specific binding between a target and its corresponding capture molecule results in a signal reflecting the presence and identity of a specific microorganism. Preferably, said hybridization is obtained under stringent conditions (well-known to persons skilled in the art).

After hybridization, target sequences can be detected by current techniques. Without labeling, preferred methods are the identification of the target by mass spectrometry or by intercalating agents followed by fluorescent detection.

Advantageously, the target sequences to be detected are labeled prior to hybridization. Said labeling (well-known to persons skilled in the art) is preferably done during amplification, if an amplification step is pursued. During amplification, labeling may be obtained using either labeled primer(s) or by incorporation of labeled nucleotide(s). Labeling can also be obtained by ligating a detectable moiety onto the target to be tested. Target sequences can also incorporate labeled nucleotides at their 5' or 3' ends using a kinase, a transferase or a similar enzyme.

The most frequently used labels are digoxigenin or fluorochromes like e.g. Cy3, Cy5 or Cy7. Radioactive, non-radioactive and cold labeling as well as indirect labeling are common methods. The resulting signal of target fixation to the capture probes may be, but is not limited to be, fluorescent, colorimetric, diffusion, electroluminescent, bio- or chemiluminescent, magnetic, electric like impedometric or voltametric. A preferred method is based upon the use of chemiluminescence, which can be easily detected and quantified by several commercially available imaging systems and software programs.

Quantification has to take into account not only the hybridization yield and detection scale on the array but also the extraction, the amplification (if any) and the labeling steps. The method according to the invention comprise means for obtaining a quantification of target nucleotide sequences by using a standard nucleic acid (exogenous and/or endogenous) added at known concentration. A capture molecule is also present on the array so as to fix the standard in the same conditions as said target (possibly after amplification). The method comprises the step of quantification of a signal resulting from the formation of a double stranded nucleotide sequence formed by complementary base pairing between the capture molecules and the exogenous and/or endogenous nucleic acids, and the step of a correlation analysis of signal resulting from the formation of said double stranded nucleotide sequence with the signal resulting from the double stranded nucleotide sequence formed by complementary base pairing between capture molecule(s) and the target in order to quantify the presence of the orginal nucleotide sequence to be detected, identified and quantified in the biological sample.

Detection of different sequences can be advantageously performed on a same array according to the present invention. In order to obtain an array providing accurate detection, identification and quantification of signals, said array contains preferably, at least three categories of capture molecules comprising,
- a first category of capture molecules being specific to target molecules,
- a second category of capture molecules being specific to control (exogenous and/or endogenous) molecules,
- a third category of capture molecules comprising labeled molecules for calibration of hybridization signals, and
- optionally a fourth category of capture molecules being specific to a group of organisms or a subdivision thereof.

Preferably, the array contains at least four categories of capture molecules as enumerated and defined above. According to the present method, detection, identification and quantification of multiple, different targets can be advantageously performed on the same array, i.e. by hybridization of targets with a large number of target-specific capture molecules.

To ensure accurate detection, identification and, more particularly, quantification of targets, advantageously, the array comprises capture molecules to specifically detect exogenous and/or endogenous control molecules. The method comprises the step of quantification of a signal resulting from the formation of a double stranded nucleotide sequence formed by complementary base pairing between the capture molecules and the exogenous and/or endogenous nucleic acids, and the step of a correlation analysis of signal resulting from the formation of said double stranded nucleotide sequence with the signal resulting from the double stranded nucleotide sequence formed by complementary base pairing between capture molecule(s) and the target in order to quantify the presence of the original nucleotide sequence to be detected, identified and quantified in the biological sample.

For calibration of hybridization signals, the array comprises reference molecules e.g. labeled molecules. Advantageously, these reference molecules are spotted from known concentrations solutions and their signals allow the conversion of the results into absolute amounts. They also allow testing for the reproducibility of the detection system.

To measure the total pool of DNA that has sequences which are complementary to the consensus primers and that thus competes with target DNA amplification, the array further comprises non-discriminative capture molecules specific to a group of microorganisms. Preferably, these capture probes are spotted at various amounts to allow addressing of possible underestimation of the target. In one embodiment, the inventors demonstrated that, under the conditions used (example 2), a specific fungal target was determined to be underestimated when a signal was obtained for a non-discriminative fungal capture probe spotted on a nylon membrane at an amount of 0.02 fmol.

It is clear from the present invention that hybridization signal intensity can be determined (quantified) using several commercially available imaging systems and software programs.

In a next step, a correlation analysis of quantified hybridization signals with microorganisms' biomass allows accurate quantification of microorganisms' biomass in a matrix or environment.

This part of the invention was made possible by showing that target sequences can be discriminated from other homologous ones upon an array with high sensitivity by using bound capture nucleotide sequences composed of preferentially two parts, one being a spacer bound to the support and the other part being a specific nucleotide sequence able to hybridize with the target sequences. Said detection is greatly increased, if high amounts of capture nucleotide sequences are spotted on the solid support. However, it will be clear from the present invention that the amount of spotted capture molecules on a solid support is dependent on many factors, including the type of hybridization system, solid support, e.g. a membrane, glass, chips, etc... Preferably, said capture molecules are spotted at an amount that allows accurate quantification of biomass. In an example, for use in a macro-array consisting of a nylon membrane loaded with bound capture molecules, the amount of capture molecules comprises more than 0.5 fmol, more than 0.8 fmol, more than 2.0 fmol, and preferably more than 5.0 fmol of capture molecules / spot.

In a further embodiment, the present invention relates to the use of an array comprising a solid support as defined herein having capture molecules bound there on for the detection, identification and quantification of multiple microorganisms or components thereof in a matrix or environment, preferably associated with agriculture and horticulture, wherein said array contains at least three and preferably at least four categories of capture molecules as defined herein.

The method according to the present invention further comprises the step of adjusting the obtained hybridization signals with a suitable correction factor. The amplification of exogenous and/or endogenous control molecules and the subsequent detection and quantification of a signal resulting from the specific binding between said exogenous and/or endogenous DNA and their corresponding specific capture molecules on an array permits monitoring amplification efficiency. Based on experimental findings, it is expected that PCR efficiencies appear quite stable between different samples. However, if the obtained signals for the standard probes are weaker than those obtained for an efficient PCR reaction, this is an indication that the amplification step was sub-optimal and that all other obtained hybridization signals, obtained in this assay, need to be corrected. The present method provides a correction of the hybridization signals with a suitable correction factor, which is determined based on the difference in signal intensity between a signal for the capture probes for the exogenous and/or endogenous standard DNA resulting from an efficiently and an non-efficiently performed PCR reaction.

Based on a single signal, a specific microorganism can be detected, identified, and quantified from a biological sample. To increase specificity, multiple capture molecules for one taxon may be spotted.

### Diagnostic kit for detecting, identifying, and quantifying microorganisms

In a further embodiment, the present invention also relates to a diagnostic kit for the detection, identification and quantification of one or more microorganisms in a matrix or environment comprising
- suitable means and media for isolating targets, e.g. genomic DNA, genomic RNA, or mRNA from said sample;
- a set of primer pairs comprising at least one consensus primer pair for amplifying target molecules, preferably DNA, from a specific group of microorganisms, and at least one primer pair for amplifying exogenous and/or endogenous control molecules, preferable control DNA, and
- an array preferably containing at least three categories of capture molecules as defined herein that are spotted on a solid support.

The method according to the invention can be performed by using a specific detection, identification and quantification system comprising at least a solid support upon which at least three and preferably at least four categories of single stranded capture molecules are bound, preferably to the surface of the solid support by a direct covalent link or, more preferably, by an intermediate spacer, according to an array with a density of at least 1, preferably at least 5, 10, 16, 20, 50, 100, 1000, 4000, 10 000 or more, different single stranded capture molecules, with said single stranded capture molecules having advantageously a length comprised between about 15 and about 500 bases, and preferably between about 18 and 300 bases, being specific to a target (which means that said bases of said sequence are able to form a binding with their complementary bases upon the sequence of the target by complementary hybridization).

The method and kit according to the invention allow easy detection, identification and quantification of specific target sequences among other possible amplified nucleotide sequences. The array may contain capture molecules for several individual organisms and/or varieties, subspecies, species, genera, families, orders, subclasses, classes and/or other groups of organisms.

The method and kit according to the invention are particularly suitable for the detection, identification and quantification of target, such as nucleic acids, being specific for a microorganism or a component thereof, possibly present in a biological sample. Said target nucleic acids can be amplified by consensus ((sub)group-specific) primers so that the identification of the target nucleotide sequence is obtained specifically by the discrimination following its binding with the corresponding capture molecule, previously bound at a given location upon a solid support.

The kit according to the invention may also incorporate various media or devices for performing a method according to the invention. Said kit can also be included in an automatic apparatus such as a high throughput screening apparatus for the detection, identification and/or the quantification of multiple nucleotide sequences present in a biological sample to be analyzed. Said kit or apparatus can be adapted for performing all the steps or only several specific steps of methods according to the invention.

Said exogenous and/or endogenous control is also advantageously included in the kit according to the invention, possibly with all the media and means necessary for performing the different steps according to the invention (hybridization and culture media, polymerase and other enzymes, primer sequence(s), labeling molecule(s), etc.).

### Apparatus

In another embodiment, the present invention also covers an apparatus necessary for performing at least some steps of the method for detecting, identifying and quantifying one or more biological organisms or component(s) thereof possibly present in a sample. Said device preferably comprises:
- capture molecules being bound to a solid support at specific locations according to an array, said array having, preferably, at least four categories of capture molecules including capture molecules being specific to target molecules, preferably target DNA; capture molecules being specific to a group of organisms ; capture molecules being specific to exogenous and/or endogenous control molecules, preferably control DNA; and reference capture molecules comprising labeled molecules for calibration of hybridization signals;
- means for detecting, identifying and quantifying a signal formed at the location of the binding between said target with said capture molecule
- means for reading information recorded upon said solid support,
- a computer program for recognizing discrete regions bearing the targets and their locations
- means for correlating the presence and intensity of a signal at these locations with the detection, identification and quantification of the said microorganisms or component(s) thereof.

In a particular embodiment, this apparatus also performs target amplification performed previously or in real time together with the detection, identification and quantification of a microorganism or its components.

The kit or apparatus according to the invention may also incorporate various media or devices for performing the method according to the invention. Said kit (or apparatus) can also be included in an automatic apparatus such as a high throughput screening apparatus for the detection, identification and the quantification of multiple DNA or RNA sequences present in a biological sample to be analyzed. Said kit or apparatus can be adapted for performing all the steps or only several specific steps of the method according to the invention.

The method according to the invention is particularly suitable for the detection identification and quantification of a target which is made of DNA or RNA. However, it is clear that the present method can be easily adapted by a person skilled in the art for a specific detection, identification and quantification of other components of biological organisms such as proteins, metabolites, receptors, antibodies, enzymes, etc...

The present invention will be further described in detail in the following non-limiting examples in reference to the enclosed figures and tables.

### Examples

### Example 1: System for assessing and reporting a risk of disease development, contamination, and loss caused by one or more microorganisms in a matrix or environment

This example illustrates a preferred embodiment of a system for assessing and reporting a risk of disease development, contamination, and loss caused by one or more microorganisms in a matrix or environment. The system is schematically illustrated in **Fig. 1** and discussed into more detail hereunder. The system comprises in block 1 data acquisition means. The data acquisition means comprises data regarding said one or more microorganisms and data regarding said matrix or environment as shown in block 11. Said data has been retrieved by direct evidence of microorganisms as indicated in block 11, i.e. by sample submission as indicated in block 111 and by a detection, identification and quantification analyses as indicated in block 112. The detection, identification and quantification method is not considered to be limitative and may comprise a DNA-based array analysis, or a RNA-or other DNA-based analysis, or protein and immunological assays, or classical culture-dependent and microscopy-based methods. Preferably, said method comprises a DNA-based analysis, as defined in the present invention. The data regarding said one or more biological organisms and said matrix or environment is also retrieved as indicated in block 12 by extensive case, which may include: information regarding past seasons or years, as indicated in block 121, information regarding present season or year, as indicated in block 122 and/or information regarding expected / future factors, as indicated in block 123. The data acquired in block 1 is communicated to an intelligent disease and contamination risk analysis and assessment unit, as indicated in block 2. Said unit 2 substantially includes an adaptive knowledge database as indicated in block 21 and analytical tools, as indicated in block 22. The adaptive knowledge database preferably is a database as defined herein including data initially assembled with conventional knowledge as shown in block 211, which is under continuous adaptation, expansion and evaluation as shown in block 212. The analytical tools as shown in block 22 may comprise data assembled by direct evidence-based threat detection as shown in block 221, disease or contamination forecast models as shown in block 222, and risk factor analyses as shown in block 223. The data acquired in block 1 is evaluated by means of the risk analysis and assessment system of block 2. Results of this risk analyses are assessed and outputted in a report by means of a risk reporting system, as indicated in block 3. Said system 3 provides a status repot as shown in block 31, a risk analysis report shown in block 32 and also a recommendation report, as shown in block 33, representing all possible options to prevent, preempt, correct or terminate existing or anticipated contamination of epidemic. The different elements of this system will be discussed into more detail hereunder.

### Element I: Situation data acquisition

In block 1 the system comprises situation data acquisition means. This element of the present invention assembles and provides situation-specific information required for the *intelligent disease and contamination risk assessment* stage, represented in block 2, by combining direct evidence of microorganism presence in a matrix or environment as represented in block 11, i.e. results from an array-based analysis as described in another embodiment of the invention or other, molecular or conventional, diagnostic approaches, with a extensive case study as represented in block 12 featuring all information relevant to the matrix or environment under examination.

### Direct evidence of microorganisms

presence, as indicated in block 11. Therefore, *"microorganism data"* refers to data received from detection, identification and density determination of microorganisms in samples during this direct evidence acquisition step. Appropriate scale, distribution, frequency, replication, and method of sampling are prescribed and followed according to each plant, crop, product, or environment under examination to assure fidelity of microorganism data reporting.

### Extensive case study

In another block, block 12, of the situation data acquisition, data beyond direct evidence of microorganism presence are collected. This data will later be effectively combined with microorganism data to achieve robust risk analyses. An extensive case study as represented in block 12 is performed featuring all information relevant to the matrix or environment under examination. "*Case study information*" refers to information about and attributes of the circumstances associated with the matrix or environment under evaluation. Case study information is collected into three areas: 1) previous activity or time period information as indicated in block 121, 2) current time or activity period considerations and observations as indicated in block 122; and 3) expected or future factors as indicated in block 123. The temporal nature of these area designations reflects the inherent chronological relevance of the information categorized and applied within. The following are non-limitative examples of the structure of these information areas:

### Previous activity or time period information e.g., information from past years, seasons, crops, production cycles, distribution activities, etc. (block 121) may include:

- Plant culture, crop production, or product manufacturing or handling methods
- Crop or production yields and losses
- Land, facilities and equipment uses and modifications
- Disease, contamination, and pest events and management actions
- Weather patterns and major meteorological events
- Chemical, physical, nutritional, and other inputs

### Current activity or time period considerations and observations e.g., information about the present situation of the matrix or environment under evaluation gathered at the start or at points throughout the current season, year, production cycle, activity, etc.) (block 122) - may include:

- Crop or specific plant variety
- Plant culture, crop production, or product manufacturing or handling methods
- Chemical, physical, and nutritional status
- Weather conditions (either single or continuous observations)
- Adjacent plants, and land features
- Site topography
- Sources, composition, and other input attributes
- Government regulations
- Economic or material value and foreseen production costs
- Potential economic return for sales of or services related to the crop, matrix or environment

### Expected or future factors (block 123) may include:

- Decision to plant, produce crop, or manufacture or handle product
- Crop selection and rotation (including varieties)
- Culture, production and handling methods
- Proximal land and facilities use and management
- Government policy and regulation
- Emerging disease, contaminant and pest threats
- Etc.

Data and information acquired during this stage of the system is referred hereafter as *"client data."* Each client data set is then deposited and archived for each client in a relational database. This database is accessible to all aspects of the risk assessment and reporting system. Client data in this database is updated and corrected as more or better information becomes available either by continued direct data acquisition e.g., meteorological observations, disease progress data, or process parameter reporting) or by feedback provided by other elements of the system. In addition, its temporal reference status in the database is changed e.g., *current activity or time period considerations* and *current microorganism data* shift to *previous activity or time period information* status with the passing of time i.e., crops, seasons, production runs, distribution cycles, years, etc..

### Element II: Intelligent disease and contamination risk assessment

The foundation of the risk assessment process of the proposed invention is composed of an ***adaptive knowledge base*** as shown in block 21 and an extensive set of *analytical tools* as shown in block 22. The *adaptive knowledge base* is central to the analytical functions and reporting of the invention. It contains conventional, published knowledge of: pathogen, contaminant, beneficial, and non-descript microorganism taxonomy, biology, host range, physiology, known interactions or associations with other organisms and various environments, survival, and other information; host or crop attributes such as but not limited to resistance and susceptibility to infection, needs, limitations, alternatives, etc.; disease symptomology, etiology and epidemiology; non-infectious or abiotic diseases , their symptoms and their causes; crop management practices; crop handling and processing; geographic disease or contamination issues and climatic conditions; matters of scale and spatial distribution of pathogens and contaminants as well as beneficial and other microorganisms; available detection, identification, and density determination methods and data scoring references; available and legal disease or contamination management options; and government regulations. This knowledge base is continually adapted, expanded, and evaluated to provide the best and most recent information possible for use by the analytical tools and the risk reporting system. Hence the adaptive nature of the knowledge base is dependent on: addition of new or emerging pathogen, disease, and contamination threats; periodic expansion to include new crops and newly threatened geographic areas; expansion, update, and correction of existing knowledge base entries based on availability of the most recent proven information; feedback or input of results from prior assessments and client outcomes (feedback from the invention); discovery of new threats by use of the invention (feedback from the invention); addition of new and optimization of existing disease and contamination management options (external information and feedback from the system); and modification or elimination of management options due to legislation, regulation, or loss of efficacy/viability.

The adaptive knowledge base is utilized for guiding the sorting, filtering, and prioritizing client data for risk assessment by the analytical tools of the system. In addition, the adaptive knowledge base is to guide application of client data to the proper analytical tools, such as but not limited to threat detection thresholds, forecast models, and risk factor analyses. The adaptive knowledge base of the present invention also serves to supply the *risk reporting* stage of the invention with specific information about named disease or contamination risk management options. Oversight and maintenance of the adaptive knowledge base is provided by a resource administrator, while regular evaluation, customization, optimization, and validation of the knowledge base is carried out by a team of professionals (a.k.a. ***professional oversight team*)**at least comprised by a plant health specialist (i.e., a plant pathologist), a molecular biologist, and a process-integrated microbiologist.

The *analytical tools* component of the invention is composed of three different yet overlapping categories of tools. ***Direct evidence-based threat detection,*** as indicated in block 221, the first of these categories, relies upon actual detection and/or density determination of pathogens, contaminants, or beneficial microorganisms and comparison of this information with information in the knowledge base to establish the degree to which a threat exists for disease or contamination of a matrix or environment. Due to its true multiplex nature, speed, objectivity, economy, and accuracy, array-based technologies such as DNA Multiscan are the most suitable sources of data for use in this category. However, results from other diagnostic methods may also be applied to verify, buttress, or further define threat determination.

***Disease or contamination forecast models,*** as indicated in block 222, is the second category of *analytical tools* and pertains to analytical processes that rely on obtaining regular lower-level detection data and / or accumulating or continuous climate or microclimate data to model and infer likelihood of infestation, disease, contamination, and loss. These analytical processes shall utilize existing validated forecast models and will include other new models or updates to existing models as the inventors or others devise them.

***Risk factor analyses,*** as indicated in block 223, are conducted for the plant, crop, plant-derived product, matrix, system, or environment under examination based on results obtained from analyses conducted in the previous two categories (i.e., *direct evidence-based threat detection* and *disease or contamination forecast models*). In the context of the invention, "*risk*" refers to the probability of incurring substantial disease, contamination, and / or economic or material loss will occur as a result of exposure to, presence of, and / or favorable conditions for development of pathogenic or contaminating microorganisms. Risk, therefore, is returned as rank value based on multiple factors including but not limited to: 1) client case study information; 2) plant, crop, matrix, or environment information (knowledge base references); 3) microorganism factors (knowledge base references); 4) microorganism data (client-specific); and 5) time. Hence, this third category of tools in the *intelligent disease and contamination risk assessment* stage of the invention compiles the results of the *direct evidence-based threat detection* and *disease or contamination forecast models* and aligns them with established criteria for various levels or magnitudes of disease, contamination and loss registered in the knowledge base to determine individual and multiple risks of diseases, contamination events, and losses. Risks will be determined using appropriate time references (i.e., immediate, seasonal, yearly, extended term, or long term).

All determined risks are then evaluated by a trained and experienced professional diagnostic plant pathologist and process-integrated microbiologist for completeness and errors. The risk factor analysis software is designed to flag or highlight unusual or unexpected results or associations including findings of no cause for disease, contamination or loss and can only accomplish this by consultation with the knowledge base reference information. It should be noted that if an existing diseased, contaminated, or other compromised condition of a plant, crop, matrix, or environment cannot be determined through the risk analysis stage, then one or all members of the professional oversight team will review results and determine whether microorganism data acquisition must be repeated (either with the same or different approaches), additional analyses are needed, or a manual risk assessment with the present client information should be performed. Once risk assessment is completed and approved by one or more members of the professional oversight team, then the resulting risk assessment is transmitted to the *risk reporting system* element of the invention, as indicated in block 3.

### Element III: Risk reporting system

This element functions as the intuitive communication mechanism of the invention. The end-product of this element is a ***client report*** communicating 1) the current status of the situation, 2) a simplified disease, contamination, or loss risk profile, and 3) a complete set of recommended actions for managing these risks.

The *risk reporting system* works in the following sequential steps:
- Retrieval of all specific client information (i.e., microorganism data and relevant case study information) and formatting of this information into a *status report* as indicated in block 31.
- Ranking of individual results of the risk factor analyses for all possible diseases and contaminants according to for example a 5-point, color-coded ranking system where blue = no risk and red = most severe risk
- Prioritization of reported risks from greatest to lowest risk as well as from most immediate to most distant concern (a temporal scale dependent on the plant, crop, matrix, or environment, and pathogen, disease, or contaminant)
- Generation of recommended actions, as indicated in block 33, to manage risks based on but not limited to level of risk, immediacy of concern and possible treatment window, client case study information, availability and efficacy of treatment strategies, costs and benefits of treatment, and likelihood of treatment success. These recommendations are derived by cross referencing client risk assessments with management strategies in the knowledge base that are most appropriate for the level and type of disease or contamination risk as well as the plant, crop, matrix, or environment under evaluation. Recommended actions may come in the forms of preventive measures, preemptive treatments, corrective or curative strategies, or termination, removal, or elimination of plant, crop, matrix, or environment or activities related to these subjects.
- Organization and presentation of the above information into a complete, concise, and understandable report on paper and in electronic form. The client then receives one or both types of reports, while the diagnostic center and knowledge base (if separate) each receive the electronic version for future reference.

Though not essential to the functionality of the present invention, the decision(s) and action(s) of the client and the outcome(s) of the action, this information is desirable for the continued evaluation and improvement of the invention as it provides feedback on the reliability of invention-derived analyses, applicability and efficacy of various management options, and client satisfaction, while facilitating updates and fine-tuning or "training" of the *adaptive knowledge base.* It is expected that client decisions, actions and outcomes will more often than not be integrated in the present invention.

### Example 2: Quantitative assessment of phytopathogenic fungi in various substrates using a DNA macroarray

The present example illustrates pathogen quantification using DNA macroarrays. In this example a format of the previously designed DNA array (Lievens et al. 2003b), is described which has been further developed for accurate quantification of the economically important vascular wilt pathogens *Verticillium albo-atrum* and *V. dahliae* for concentration ranges typically encountered in horticultural practice.

### MATERIALS AND METHODS

**Microorganisms and cultivation.** The fungal isolates *Fusarium oxysporum* f. sp. *lycopersici* CBS 646.78, *F. solani* CBS 165.87, *V. albo-atrum* CBS 451.88, *V. dahliae* CBS 381.66, *Pythium ultimum* CBS 656.68 (Centraalbureau voor Schimmelcultures, Utrecht, The Netherlands), and *P. aphanidermatum* ST 59.04 (Scientia Terrae Research Insititute, Sint-Katelijne-Waver, Belgium) were cultured on potato dextrose agar (PDA). Saccharomyces cerevisiae MUCL 28426 (Mycothèque de l'Université Catholique de Louvain, Louvain-la-Neuve, Belgium) and the bacterial strain *Rhizobium vitis* LMG 258 (Laboratory of Microbiology, Ghent University, Gent, Belgium) were cultivated on malt extract agar supplemented with yeast extract (2%) and nutrient agar, respectively. All cultures were incubated in darkness at 24°C.

**Production of artificially inoculated samples**. To quantify pathogen occurrence using DNA arrays, different samples were produced containing biologically relevant pathogen concentrations. Initially, microsclerotia from *V. dahliae,* or conidia from either *V. albo-atrum* or *V. dahliae* were added to 0.75 g of sandy field soil. These samples were subsequently used for DNA extraction and DNA array analysis. Microsclerotia were produced according to the method described by Hawke and Lazarovits (Hawke and Lazarovits 1994 Phytopathology 84:883-890). The microsclerotia were suspended in sterile distilled water, vortexed briefly to disrupt microsclerotia aggregates, washed through a 125-µm sieve with sterile distilled water, and collected on a 32-µm sieve. Conidia were obtained by gently washing a culture plate with sterile distilled water. Conidial cells were counted by direct light microscopy using a haemocytometer, serially diluted, and adjusted to the desired concentration. In addition, water-based samples were collected for quantitative assessment of the tomato pathogen P. *aphanidermatum.* Zoospores were produced in 20 ml sterile mineral salt solution (0.68 mM Ca2+, 0.05 mM Mg2+, 0.73 mM K+, and 0.06 Fe3+) inoculated with 10 agar plugs (05 mm) of P. aphanidermatum grown on V8 agar. After 1 day of incubation at 24 °C under continuous illumination, zoospores were harvested and counted as described above. Next, ten 14-day old tomato (*Lycopersicon esculentum* Mill. cv. Clotilde) seedlings were transferred to test tubes filled with 6 ml of nutrient solution (Cooper 1979 Grower Books, London, UK) containing 102, 103 or 104 zoospores/ml. Plants were incubated in a growth chamber with a 16-h photoperiod (225 µE/m2/s) at 22°C. After 10 days, disease severity rating (DSR) for plant root and foot rot was scored on a 1 to 5 scale: 1 =symptomless, 2=light browning and/or superficial lesions present, 3=dark browning and/or sunken lesions present, 4=development of coalescing lesions and necrosis, 5=plant death. At the same time, the remaining nutrient solution was collected and used for DNA extraction and DNA array analysis.

**Collection of soil samples**. To define the range of relevant DNA concentrations by which soilborne pathogens occur in their natural habitats, several soil samples were collected from commercially exploited greenhouses at various times during the growing season. Subsamples were retained for DNA extraction. Separately, soil samples were collected from two fields that were naturally infested with *V. dahliae* and air-dried for two weeks. The number of viable *V. dahliae* microsclerotia was determined using the wet sieving technique (Harris et al. 1993 Plant Pathol. 42:238-250). Briefly, 12.5 g of air-dried soil was wet sieved, followed by suspending the 20-100 µm fraction in 0.08% agar. Subsequently, 0.8 ml of this suspension was spread on modified soil extract agar medium (Harris et al. 1993). Plates were incubated in darkness at 24°C. After 4 weeks, soil particles were removed from the plates and clusters of *Verticillium* microsclerotia were counted. In addition, subsamples were retained for DNA extraction.

**DNA extraction**. Genomic DNA from all microorganisms was isolated as previously described (Lievens et al. 2003b). For DNA isolation from soil and plant samples, genomic DNA was extracted from 0.75 g starting material using the Ultraclean Soil DNA Isolation Kit and the Ultraclean Plant DNA Isolation Kit as described by the manufacturer (Mo Bio Laboratories, Inc., Solana Beach, CA, USA), and subsequently diluted 10 times. For water-based samples, DNA was extracted using the Ultraclean Water DNA Isolation Kit (Mo Bio Laboratories, Inc., Solana Beach, CA, USA) as described by the manufacturer's protocol. DNA yields were determined spectrophotometrically at 260 nm.

**DNA array production**. The taxon-specific detector probes used in this example were previously selected (Lievens et al. 2003b) and are listed in table 1. In addition to the previously used control oligonucleotides Uni1 and Con1, as a control for the hybridization, and Dig1, as a reference for the detection and calibration (Lievens et al. 2003b), an oligonucleotide was designed to target exogenous control DNA derived from bakers yeast (Sce1). All oligonucleotides were synthesized with a 5'-C6-amino linker for covalent binding to nylon membrane and were diluted in sodium bicarbonate buffer (0.5 M, pH 8.4) in a microtiter plate and spotted in duplicate on Immunodyne ABC membranes (PALL Europe Limited, Portsmouth, UK) using a multi-blot replicator (V&P Scientific, Inc., San Diego, CA, USA). For this example, the detector probes Fun1, Fox2, Fso1, Val2, and Vda1 were spotted on the membrane at different quantities, namely 8.0, 2.0, 0.5, 0.2, 0.1, and 0.02 fmol. For the control oligonucleotides, 8.0 fmol (Uni1, Con1, Sce1) or 2.0 (Dig1) fmol was printed on the membrane. After a 10-min drying step, blots were transferred into blocking solution (2x standard saline citrate (SSC), 0.5% casein, and 0.05% Tween 20) and agitated for 30 min at room temperature. Membranes were stored in 2x SSC at 4°C until use.

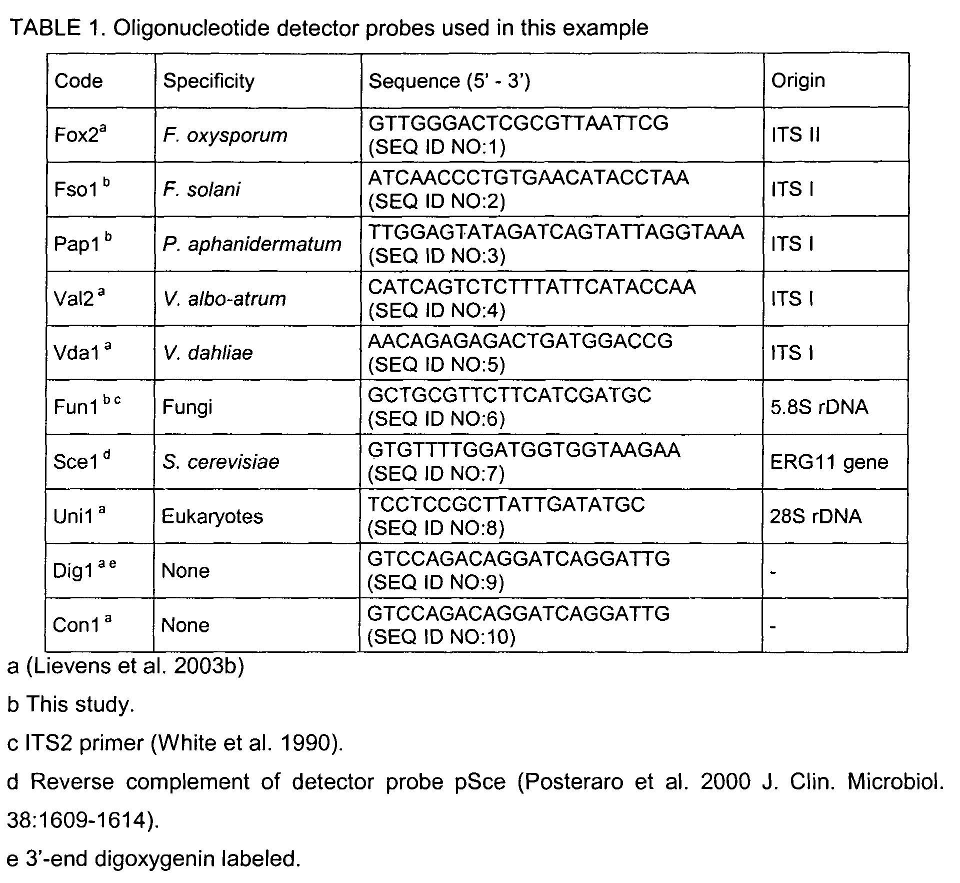

**PCR amplification and labeling**. The target internal transcribed spacer (ITS) region of fungal rDNA was amplified using the primers ITS1-F and ITS4 (Gardes and Bruns 1993 Mol. Ecol. 2:113-118). Amplification was carried out in 20 µl reaction volume using 1 unit Titanium Taq DNA polymerase (Clontech Laboratories, Inc., Palo Alto, CA, USA) according to the manufacturer's protocol. Prior to amplification, samples were preheated to 94°C for 2 min. Next, 30 cycles of a PCR reaction protocol consisting of 45 s at 94°C, 45 s at 59°C, and 45 s at 72°C, with a final 10-min extension step at 72°C were run. After gel electrophoresis, PCR products were quantified by comparison to a DNA ladder using Labworks Image Acquisition and Analysis Software (version 4.0; UVP, Inc., Upland, CA, USA). For DNA array analysis, the target ITS region was amplified and simultaneously labeled with alkaline-labile digoxigenin (Roche Diagnostics GmbH, Mannheim, Germany) using the fungal primer set ITS1-F and ITS4 (Gardes and Bruns 1993) or the oomycete primer set OOMUP18Sc and ITS4 (Lievens et al. 2004 Plant Dis. 88:86). As a control for PCR efficiency between different samples, an exogenous control DNA fragment from S. cerevisiae was co-amplified and labeled using primers P4501 and P4502 (Morace et al. 1997 J. Clin. Microbiol. 35:667-672). The target samples were amplified in 20 µl using 1 unit Titanium Taq DNA polymerase in the presence of 7.5 µM digoxigenin-11-d-UTP (Dig-dUTP; Roche Diagnostics GmbH, Mannheim, Germany) and 1 ng external control DNA according to the same thermal profile as described above. The resulting Dig-dUTP-labeled amplicons were subsequently used as targets in hybridization reactions to the array.

**DNA Hybridization of PCR amplicons.** Hybridization was conducted as described previously (Lievens et al. 2003b). Ten µl labeled amplicons were hybridized to the array in 6 ml hybridization buffer. Chemiluminescence was detected cumulatively at 30 s intervals over 45 min using a highly sensitive digital CCD video imaging system (BioChemi System; UVP, Inc., Upland, CA, USA). Quantification of hybridization signals was carried out using Labworks Image Acquisition and Analysis Software. Hybridization signal strength was reported relative to the average integrated optical density of the digoxigenin-labeled reference control. All hybridization assays were conducted at least twice.

**Real-time quantitative PCR**. To define the range of relevant DNA concentrations by which fungal pathogens occur in naturally infested greenhouse soils, the amount of fungal DNA was quantified in a set of representative soil samples using real-time PCR. This was done for a number of different pathogens detected in these samples using the DNA Multiscan® (De Ceuster Corp., Sint-Katelijne-Waver, Belgium). Real-time PCR amplification reactions were conducted using SYBR® Green I technology on a Lightcycler™ instrument (Roche Diagnostics Corp., Indianapolis, IN, USA). Each reaction mixture contained 2 µl DNA extract, 4 µl of the Lightcycler FastStart DNA MasterPLUS SYBR® Green I kit (Roche Diagnostics Corp., Indianapolis, IN, USA), 1 µl of each primer (10 µM), and 12 µl sterile distilled water. For each pathogen, the forward primer ITS1-F (Gardes and Bruns 1993) or OOMUP18Sc (Lievens et al. 2004), which hybridizes to a fungal- or oomycete-specific rDNA sequence respectively, or the universal reverse primer ITS4 (White et al. 1990 In M. A. Innis, D. H. Gelfand, J. J. Sninsky, and T. J. White (ed.), PCR protocols, a guide to methods and applications. Academic Press, San Diego, CA, USA) was combined with the appropriate reverse or forward species-specific primer as presented in table 2, to generate amplicons smaller than 300 bp. The amount of total fungal and oomycete DNA was quantified using the primer pair ITS1-F and ITS2 (White et al. 1990), and OOMUP18Sc and ITS2-O (table 2), respectively. Samples were preheated to 95°C for 10 min and were then subjected to PCR amplification reactions consisting of 45 amplification cycles of 10 s at 95°C, 5 s at the annealing temperature indicated in table 2, and elongation at 72°C for the time period indicated in table 2. Fluorescence (520 nm) was detected at the end of the elongation phase for each cycle. After the final amplification cycle, a melting curve temperature profile was obtained by heating the mixture to 95°C, cooling to 65°C (15 s) and slowly heating to 95°C at 0.1 °C s-1 with continuous measurement of fluorescence at 520 nm. For each pathogen, standard curves were generated by plotting the threshold cycle (Ct) of a 10-fold dilution series of genomic DNA versus the logarithm of the concentration. The regression line was used to calculate the respective pathogen DNA concentration in the studied sample via its Ct- value.

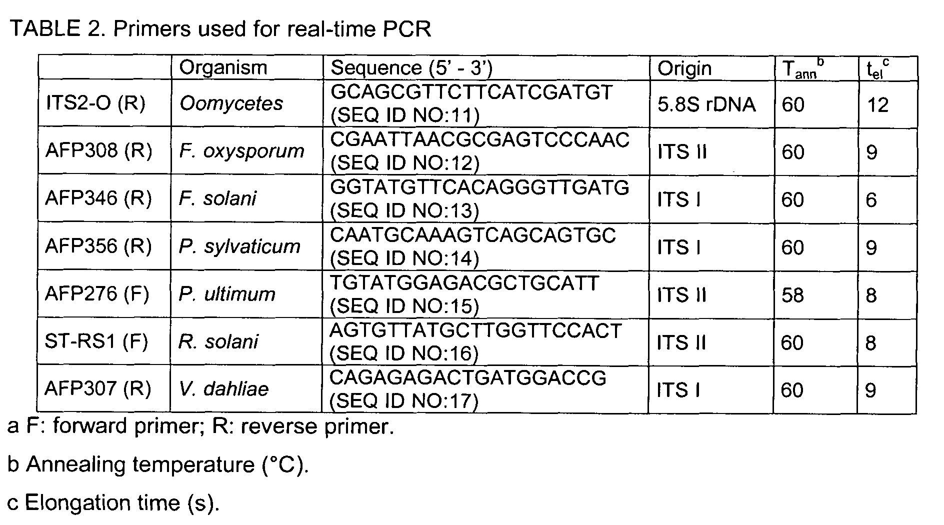

### RESULTS

**Optimizing PCR conditions to permit end-point quantification**. For sensitive pathogen detection using DNA arrays, PCR pre-amplification is required. There are, however, limitations to the use of PCR in a quantitative approach, since bias in template-to-product ratios may be introduced due to typical PCR amplification kinetics (Suzuki and Giovannoni 1996). As a result, the dynamic range of the targets to be detected may not always be reflected by the outcome of the assay. This bias in template-to-product ratio can be caused by two technical artifacts, namely (Amannet al. 1995) differential PCR efficiency between samples, or (Anthony et al. 2000) analysis of samples which are no longer in the exponential phase of the reaction. To monitor this potential problem, an equal amount of an exogenous control DNA derived from S. cerevisiae was added to each PCR reaction to verify whether the PCR efficiency between different samples was comparable. In table 3, PCR efficiencies are shown for a 10-fold dilution series of genomic DNA from *V. albo-atrum* and *V. dahliae* to which 2.5 ng of DNA extracted from sandy greenhouse soil or healthy tomato leaf material was added prior to DNA amplification. As can be observed, PCR efficiencies appeared quite stable for all conditions tested. For all following experiments, PCR efficiencies between analyzed samples were highly comparable. In addition to adding exogenous control DNA, PCR reaction parameters were adjusted to ensure hybridization with amplicons arising from the exponential phase of the PCR reaction. To this end, a 10-fold dilution series of genomic DNA from *V. albo-atrum* (5 ng to 0.5 pg) and *V. dahliae* (25 ng to 2.5 pg) was amplified using either 25, 30, 35, or 40 cycles. PCR products were quantified by comparison to standard DNA, showing that upto 35 cycles, most PCR reactions remained in the exponential phase (table 4). In addition, genomic DNA isolated from naturally infested soils and infected plants was amplified under the same conditions, essentially showing similar results (table 4). A comparable experiment was performed for other fungal pathogens, including the tomato pathogens *F. oxysporum f.sp. lycopersici* and *F. solani.* In all cases, PCR reactions were found to be in the exponential phase upto at least 30 cycles (data not shown). Therefore, all following PCRs were performed using 30 cycles to ensure detection at the exponential phase of the PCR reaction in combination with high sensitivity.

**TABLE 3.**

| Comparison of PCR efficiencies^{a} between different samples | | | | | | |
|---|---|---|---|---|---|---|
| Origin of DNA sample (2.5 ng) | Target organism | PCR efficiencies^{a} at target DNA amounts of | | | | |
| | | 0.25 pg | 2.5 pg | 25 pg | 250 pg | 2.5 ng |
| Soil | *V. albo-atrum* | 0.85 + 0.24 | 0.93 + 0.12 | 0.79 + 0.21 | 0.84 + 0.16 | 1.07 + 0.02 |
| Plant | *V. albo-atrum* | 1.05 + 0.25 | 1.06 + 0.09 | 0.98 + 0.04 | 0.93 + 0.25 | 1.00 + 0.05 |
| Soil | *V. dahliae* | 0.95 + 0.37 | 0.96 + 0.25 | 1.07 + 0.35 | 0.98 + 0.28 | 1.05 + 0.26 |
| Plant | *V. dahliae* | 0.88 + 0.26 | 1.07 + 0.19 | 0.78 + 0.14 | 1.02 + 0.23 | 0.88 + 0.18 |

a Reported as the ratio between the hybridization signals for the oligonucleotide detector probes to target the exogenous control DNA derived from *S. cerevisiae* (Sce1) and the digoxigenin-labeled reference control (Dig1). Values are means + standard deviations (n = 4 from 2 independent analyses).

**TABLE 4.**

| Yield of PCR product (ng/µl) after a specific number of PCR cycles | | | | | | |
|---|---|---|---|---|---|---|
| Sample | Code | Template DNA (ng) | Yield of PCR product after PCR cycle number | | | |
| | | | 25 | 30 | 35 | 40 |
| *V. albo-atrum* | | 5 | 81.7 | 96.3 | 112.1 | 118.3 |
| | | 0.5 | 21.2 | 59.7 | 97.9 | 99.6 |
| | | 0.05 | 3.7 | 24.3 | 40.3 | 43.5 |
| | | 0.005 | 0.0 | 0.9 | 1.2 | 2.3 |
| | | 0.0005 | 0.0 | 0.0 | 0.0 | 0.0 |
| *V. dahliae* | | 25 | 69.8 | 377.7 | 442.9 | 441.7 |
| | | 2.5 | 14.8 | 82.0 | 218.3 | 254.7 |
| | | 0.25 | 0.0 | 19.4 | 34.0 | 34.7 |
| | | 0.025 | 0.0 | 8.4 | 12.2 | 32.9 |
| | | 0.0025 | 0.0 | 0.0 | 0.0 | 7.9 |
| Soil | 03-311 | 2.75 | 12.5 | 47.3 | 105.7 | 166.4 |
| | 03-324 | 12.75 | 20.9 | 71.6 | 67.6 | 45.4 |
| Plant | 03-312 | 1.5 | 19.1 | 48.9 | 127.3 | 178.6 |
| | P58 | 8.75 | 21.9 | 86.3 | 216.6 | 176.9 |

**Quantification of DNA dilutions using a DNA array**. One potential problem that can hamper quantification using DNA arrays is inter-spot variability caused by printing defaults or spatial effects. To test this, 8.0 fmol of detector probe Vda1 was spotted in duplicate at 6 different locations on a membrane and labeled *V. dahliae* amplicon (10 ng/ml) was hybridized to the membrane. The signals obtained showed an average relative optical density of 112.4 + 16.1, demonstrating that the inter-spot variability is limited. To investigate the quantitative properties of DNA arrays, accurate quantification of a 10-fold dilution series of *V. dahliae* genomic DNA after PCR amplification was pursued. The fungal template ranged from 2.5 ng to 0.25 pg, and 30 cycles of PCR amplification were performed. On the DNA array, different amounts of the same detector probe (Vda1) were spotted. In principal, those detector probes that show a perfect correlation between the signal intensity and the amount of template before PCR amplification will permit accurate template quantification. Hybridization results revealed that signals increased by increasing the printed amount of detector probe, especially when spotted at amounts lower than 2.0 fmol (Fig. 2A). For all spotted amounts, a linear logarithmic relationship between the integrated spot intensity and template DNA concentration could be obtained for a specific concentration range. When considering the complete concentration range, the correlation was almost perfectly linear (R2 = 0.99) when 0.5 fmol of probe was delivered per spot. In other cases, however, the curves deviate from linearity for the lower or higher DNA concentrations used in this dilution range. The latter was particularly observed when signals were strong and at the saturation level of the immobilized oligonucleotide. When 2.0 or 8.0 fmol detector probe was spotted, hybridization signals were saturated at 25 pg template DNA or more. Additions of 2.5 ng of DNA extracted from sandy greenhouse soil or from healthy tomato leaf material to the samples of the dilution series prior to DNA amplification did not influence the hybridization results (data not shown). A similar experiment was conducted for *V. albo-atrum* (Fig. 2B) as well as for other fungal pathogens, including the tomato pathogens *F. oxysporum f. sp. lycopersici* and *F. solani* (data not shown), confirming the outcome of this experiment.

**Direct quantification of fungal DNA in environmental samples using real-time PCR**. To find the most appropriate amount of spotted probe for quantification of pathogen biomass in environmental samples, the range of relevant DNA concentrations was defined for a number of different plant pathogens. Initially, 10 soil samples obtained from commercial vegetable growers at different periods during the growing season were assessed for pathogen occurrence using the DNA Multiscan®. Subsequently, for all pathogens detected, the amount of genomic DNA was quantified using real-time PCR (table 5). In addition to *V. dahliae,* pathogens detected by DNA Multiscan® included *F. oxysporum, F. solani, Pythium sylvaticum, P. ultimum* and *Rhizoctonia solani.* The tested DNA extracts contained on average 5 ng/µl genomic DNA of which between 1 and 300 pg/µl of fungal or oomycete origin. Up to 25% of this DNA turned out to be from a single pathogen. The average concentration of DNA from a single pathogen was established at 3 pg/µl and the maximum found in this assay was 12 pg/µl. Based on these findings, a detector probe amount of 8.0 fmol per spot was selected for further experiments. At this amount, detection was most sensitive and a linear logarithmic relationship was obtained for concentrations up to 25 pg/µl, which represents a realistic range of plant pathogen DNA concentrations that are relevant for naturally infested greenhouse soils.

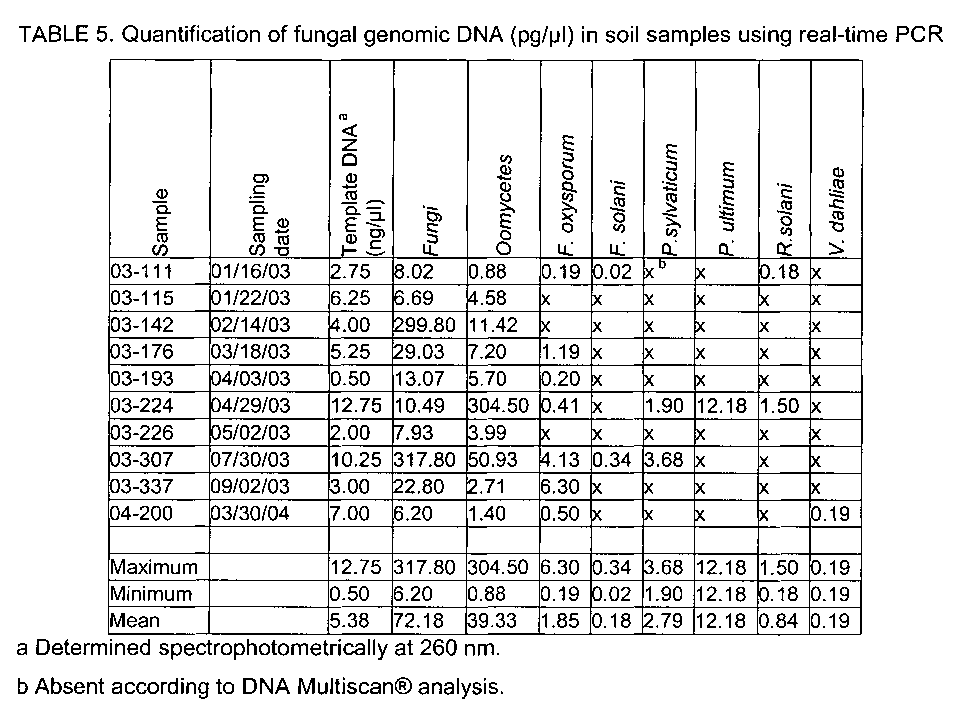

**Influence of non-target DNA on target quantification using the DNA array**. Because the ultimate goal of this work was to quantify pathogen presence in DNA extracts from complex biological materials, the possible interference of non-target DNA of different origins with accurate detection and quantification was tested. A 10-fold dilution series of genomic DNA from *V. albo-atrum* and *V. dahliae* ranging from 0.25 pg (reflecting a light or early infestation) to 25 pg (resembling a strong infestation) was amplified in the presence of a specific amount of non-target DNA. Either 25 pg, 250 pg, or 2.5 ng of non-target DNA was added to the PCR mixture, which resulted in testing pathogen:non-target DNA ratios up to 1:100, 1:1000, and 1:10000 respectively. DNA templates isolated from bacterial (*R. vitis*), oomycete (*P. ultimum*), and fungal (*F. solani*) cultures, and from healthy tomato plant and sandy soil were used. After PCR amplification, amplicons were hybridized to the array and analyzed. Fig. 3 represents a typical example of signals after hybridization, showing similar hybridization strengths irrespective the presence of 250 pg non-target DNA. Whereas signal intensities were not influenced by bacterial-, oomycete-, plant- or soil-derived DNA and in all cases as little as 0.25 pg target DNA could be detected, non-target fungal DNA affected hybridization results when present at certain ratios (table 6). In general, non-target fungal DNA did not interfere with detection and quantification up to a target:non-target ratio of 1:1000. For the lowest concentration of target DNA (0.25 pg), the highest concentration of fungal non-target (target:non-target ratio 1:10000) resulted in an inability to detect the target. In that case, increasing the number of PCR cycles to 40, however, made detection of target DNA possible. Similar experiments were also performed for *F.oxysporum f.sp, lycopersici,* which essentially provided similar results (data not shown). It can thus be concluded that this high amount of fungal non-target DNA results in an underestimation of target DNA. A non-discriminative fungal detector probe (Fun1), based on 5.8S rDNA sequences, was added to the array in order to measure the total pool of fungal DNA in the sample and thus address possible underestimation of the target. In general, if signals were obtained when this probe was spotted at an amount of 0.02 fmol, the target was determined to be underestimated (table 6).

**TABLE 6.**

| Influence of non-target fungal (F. solani) DNA on target DNA quantification | | | | | | |
|---|---|---|---|---|---|---|
| | Hybridization signals for different target DNA amounts of | | | | | |
| *F. solani* template^{a} | 0.25 pg *V. albo-atrum* | | | 2.5 pg *V. albo-atrum* | | |
| | Ratio^{b} | Val2^{c} | Fun1^{d} | Ratio | Val2 | Fun1 |
| Con | 1:0 | 3.9 + 4.4^{e} | 0.0 + 0.0 | 1:0 | 51.7 + 7.5 | 0.0 + 0.0 |
| 25 pg | 1:100 | 3.6 + 0.7 | 0.0 + 0.0 | 1:10 | 61.2 + 13.7 | 0.0 + 0.0 |
| 250 pg | 1:1000 | 1.8 + 1.1 | 0.0 + 0.0 | 1:100 | 58.9 + 12.6 | 0.2 + 0.2 |
| 2.5 ng | 1:10000 | 0.0 + 0.0 | 4.2 + 2.3 | 1:1000 | 28.9 + 25.2 | 6.6 + 2.3 |
| | | | | | | |

| *F. solani* template^{a} | 25 pg *V. albo-atrum* | | | 0.25 pg *V. dahliae* | | |
|---|---|---|---|---|---|---|
| | Ratio | Val2 | Fun1 | Ratio | Vda1^{c} | Fun1 |
| Con | 1:0 | 90.6 + 9.9 | 0.0 + 0.0 | 1:0 | 6.7 + 4.6 | 0.0 + 0.0 |
| 25 pg | 1:1 | 88.9 + 34.3 | 0.0 + 0.0 | 1:100 | 10.9 + 8.7 | 0.0 + 0.0 |
| 250 pg | 1:10 | 93.7 + 31.6 | 1.4 + 0.7 | 1:1000 | 11.5 + 1.8 | 0.0 + 0.0 |
| 2.5 ng | 1:100 | 81.7 + 30.6 | 8.6 + 5.7 | 1:10000 | 0.0 + 0.0 | 2.9 + 0.3 |
| | | | | | | |

| *F. solani* template^{a} | 2.5 pg *V*. *dahliae* | | | 25 pg *V*. *dahliae* | | |
|---|---|---|---|---|---|---|
| | Ratio | Vda1 | Fun1 | Ratio | Vda1 | Fun1 |
| Con | 1:0 | 78.8 + 9.4 | 0.0 + 0.0 | 1:0 | 116.5 + 8.9 | 0.0 + 0.0 |
| 25 pg | 1:10 | 90.9 + 21.2 | 0.2 + 0.1 | 1:1 | 121.7 + 6.3 | 0.1 + 0.2 |
| 250 pg | 1:100 | 53.9 + 21.0 | 0.0 + 0.0 | 1:10 | 127.0 + 8.9 | 0.7 + 0.6 |
| 2.5 ng | 1:1000 | 36.1 + 14.8 | 10.3 + 4.5 | 1:100 | 134.7 + 6.5 | 6.1 + 2.2 |

| | | | | | | |
|---|---|---|---|---|---|---|
| a Amount of *F. solani* DNA template in the DNA mixture. | | | | | | |
| b Target : non-target ratio. | | | | | | |
| c Hybridization to the species-specific detector probes Val2 and Vda1 (8.0 fmol) to detect *V. albo-atrum* and *V. dahliae*, respectively. | | | | | | |
| d Hybridization to the non-discriminative fungal detector probe Fun1 spotted at an amount of 0.02 fmol. | | | | | | |
| e Hybridization signal strength is reported relative to the average integrated optical density of the digoxigenin labeled reference control (Dig1). Values are means + standard deviations (n = 4 from 2 independent analyses). | | | | | | |

**Quantitative assessment of pathogen presence in artificially inoculated and naturally infested samples.** To quantify pathogen biomass in complex biological samples, soil was infested with specific amounts of conidia from either *V. albo-atrum* or *V. dahliae,* or microsclerotia from *V. dahliae.* The relationships of hybridization strength to the logarithmic number of *V. dahliae* spores and microsclerotia are presented in Figs. 4A and B, respectively, demonstrating that quantitative detection of the pathogen was successful in artificially infested mixes. A perfect linear correlation was obtained with a coefficient of determination of 0.96 and 0.99 between 103 and 106 spores or 5 and 40 microsclerotia, respectively, each representing realistic ranges by which these pathogens occur under natural circumstances (Xiao and Subbarao 1998 Phytopathology 88:1108-1115). In addition, we evaluated whether the DNA array could also be used for the assessment of fungal biomass in naturally infested soils. Based on the results shown in Fig. 4B, the relation between the hybridization strength and the number of *V. dahliae* microsclerotia is described by the regression equation y = 12.281x - 4.7315, with y being the relative integrated optical density and x representing the logarithmic number of microsclerotia. This formula was used to estimate the number of microsclerotia present in the soil of two fields exhibiting wilt symptoms. Using the DNA array, the number of microsclerotia in 0.75 g air-dried soil was estimated at 10 and 7, corresponding to 13 and 9 microsclerotia g-1 soil, respectively. With the classical wet sieving technique in both soils the number of recovered microsclerotia was established at 7 microsclerotia g-1 soil. However, since a portion of the microsclerotia generally gets lost by sieving (Goud et al. 2003), it was expected to find more microsclerotia using the DNA array than by using the classical method.

In order to generalize the data obtained in this example, we finally used these findings to pursue the development of a quantitative detector probe for a completely different pathogen. The oomycete *P. aphanidermatum* was chosen as target organism and a detector probe (Pap1) was spotted at 8.0 fmol. Tomato seedlings grown for 10 days in nutrient solution containing specific concentrations (102 to 104 zoospores/ml) of *P. aphanidermatum* zoospores were rated for foot and root rot severity (Fig. 5B). At that time symptoms of reduced plant growth were well developed. In addition, DNA was extracted from the nutrient solution for hybridization to the array (Fig. 5A). The results of this experiment showed a strong correlation between the hybridization signal intensity, the amount of zoospores, and disease severity (Fig. 5), demonstrating the feasibility of the technique to quantitatively monitor plant health and to quantitatively detect a different pathogen.

### DISCUSSION

DNA array technology is one of the most applied techniques to detect multiple microorganisms in a single assay from diverse environments (Lévesque 2001). One major shortcoming until now, however, has been its lack of quantitative character allowing the evaluation of the severity of an infestation. This has implications for interpretation of pathogen assessment surveys and decision making whether or not disease control strategies should be undertaken based on the presence of certain signals. In this example a new format of the previously designed DNA array (Lievens et al. 2003b) is described, which has been further developed for accurate quantification of the economically important vascular wilt pathogens *V. albo-atrum* and *V. dahliae* for concentration ranges typically encountered in horticultural practice.

This example shows that by including several controls, hybridization results can be standardized and accurately quantified allowing quantitative assessment of pathogen biomass. To use DNA arrays for diagnostic purposes in plant pathology, PCR preamplification is required to obtain the desired sensitivity. However, end-point quantification after PCR is often accompanied by bias in template-to-product ratio (Suzuki and Giovannoni 1996 Appl. Environ. Microbiol. 62:625-630). This ratio may be skewed by two major technical artifacts, namely variability in PCR efficiency and template saturation. Variability in PCR efficiency is generally caused by compounds in sample materials that reduce or inhibit amplification efficiency (including phenolic compounds, humic acids, fulvic acids, heavy metals, and excessive non-target DNA). In this example an equal amount of exogenous control DNA (derived from S. cerevisiae, generally not a common soil inhibitant), was added to each PCR reaction mixture. Using the detector probe Sce1 on the DNA array, PCR efficiency between samples could be monitored. In this example, PCR efficiency between all samples analyzed was highly comparable, regardless of the sample matrix from which DNA was isolated. Obviously, the quality of the DNA to be amplified is critical (López et al. 2003 Int. Microbiol. 6: 233-243; McCartney et al. 2003). Therefore, these results also suggest that high-quality purified DNA was obtained in this work by using the commercially available Mo Bio Ultra Clean DNA extraction kits.

Based on the data obtained in this example, it can be stated that a spotted probe amount of 0.5 fmol allows quantification of template DNA over a wide concentration range. In contrast, a spotted probe amount of 2.0 fmol or more allows a more sensitive detection at the lower concentrations accompanied by a loss of resolution at the higher concentrations. Thus, the choice of probe amount should depend on the range of concentrations that need to be measured, which is determined by the range of concentrations by which these pathogens are found in horticultural practice. By considering pathogen biomass and, hence, their corresponding DNA concentrations that typically occur in cultivated horticultural soils, this example shows that that accurate DNA quantification is optimal when 8.0 fmol detector probe was spotted on the membrane, irrespective the presence or absence of non-target DNA. With this amount of detector probe, the assay was determined to be the most sensitive and quantitative over a range covering at least 3 orders of magnitude that are relevant to biological and horticultural conditions. Increasing the amount of detector probe did not enhance detection sensitivity (data not shown), probably due to steric hindrance caused by the high packing of the oligonucleotides bound to the membrane. Additional experiments have revealed that certain oligonucleotides are easily saturated, even at rather low DNA concentrations. As a consequence, the choice of probe amount depends on the detector sequence and should, therefore, be determined for each probe individually.

In a previous study (Lievens et al. 2003b), we have shown that the detection limit of the DNA array technique largely depends on the detector sequences used. Generally, less than a picogram of DNA from a single target organism could easily be detected, if the appropriate detector probe sequence is used. However, based on the data obtained in this example, it can be concluded that the detection limit of a specific probe is, in addition, determined by the amount of non-target DNA from fungal origin. When the amount of fungal non-target DNA exceeded the target DNA over 1000-fold, the lowest amount of target DNA tested in this example (0.25 pg) was not detectable anymore. This discrepancy can be explained by competition between the target and non-target fungal DNA for PCR reagents, since both are amplified with the same primer pair. Experiments with other fungal detector probes show that the interference caused by fungal non-target DNA is a general phenomenon. To check for possible underestimation of target presence, a non-discriminative fungal detector probe (Fun1) was added to the membrane. In general, if signals were obtained when this probe was printed at an amount of 0.02 fmol, the target was underestimated.

Using DNA array technology, in principle, an unlimited amount of microorganisms can be detected in a single assay. The most advanced DNA array platforms are high-density DNA microarrays for which oligonucleotides are either synthesized in situ or spotted onto an impermeable rigid support such as a glass slide (Cunningham 2000 J. Pharmacol. Toxicol. Methods 44:291-300). There are a number of specific disadvantages to the use of microarrays compared to macroarrays for disease diagnostic use in plant pathology. Microarray technology is generally less sensitive than conventional membrane hybridization due to the limitations in the amount of detector probe that can be spotted on the solid support and the corresponding limit in loading capacity (Cho and Tiedje 2002 Appl. Environ. Microbiol. 68:1425-1430, Voordouw et al. 1993). As a consequence, a prior step of PCR amplification is also required for microarray technology to permit sensitive detection. In addition, quantification still remains problematic due to difficulties in spot detection and inter-and intra-spot variability (Cho and Tiedje 2002; Cuzin 2001 Transfus. Clin. Biol. 8:291-296). In contrast, inter-spot variability for the present macroarray was rather limited. Finally, highly specialized instruments are needed for microarray fabrication, as well as for hybridization and reading of the hybridization results, making implementation of this technology for plant disease diagnosis rather expensive. In contrast, a macroarray-based detection procedure for plant pathogens is affordable. Ultimately, thie present approach may lead to a complete pathogen assessment method to detect and quantify all possible pathogens (including fungi, bacteria, nematodes, as well as viruses) of specific crops.

### CONCLUSION

Detection, identification and quantification of plant pathogens are the cornerstones of preventive disease management in many crops. To detect multiple different pathogens in a single assay, DNA array technology is currently the most suitable technique. However, for sensitive detection, PCR amplification prior to array hybridization is required. This example illustrates the design and optimization of a DNA array for accurate quantification over at least 3 orders of magnitude of the economically important vascular wilt pathogens *Verticillium albo-atrum* and *V. dahliae.* A strong correlation (R2 > 0.96) was observed between hybridization signals and pathogen concentrations for standard DNA added to DNA from different origins and for infested samples. While accounting for specific criteria like probe density and controls for PCR kinetics, accurate quantification of pathogens in concentration ranges typically encountered in horticultural practice was achieved. This technique can be applied in plant pathology to analyze content of pathogens in a diversity of sample matrices including soil, water and plants.

## Claims

1. Method for assessing and reporting risks of disease, contamination or loss caused by one or more microorganisms in a matrix or an environment, said method comprising the steps of:
a) acquiring data regarding said one or more microorganisms and data regarding said matrix or said environment,
b) performing a risk analysis and assessment in order to determine a risk of disease, contamination or loss by said one or more microorganisms in said matrix or environment, and
c) compiling the results of said risk analysis and outputting said results in a report.

2. Method according to claim 1, wherein data regarding said microorganisms is at least partly acquired by detecting, identifying and quantifying one or more microorganisms or components thereof in said matrix or in said environment in an array-based assay.

3. Method for detecting, identifying, and quantifying microorganisms or components thereof in an agricultural or horticultural matrix or environment comprising:
- extracting targets from said matrix or environment,
- contacting said targets, preferably after an amplification step, with capture molecules bound to a solid support; and
- detecting and quantifying signals resulting from the specific binding between said targets and their corresponding specific capture molecules,
wherein said capture molecules are bound to a solid support at a specific location in an array format,
wherein the binding between targets and their corresponding capture molecules forms said signals at the expected location, and
wherein said signals are correlated to the detection, identification and quantification of specific microorganisms or components thereof.

4. Method according to claim 1 or 2, wherein said one or more microorganisms or components thereof are detected, identified and quantified according to the method of claim 3.

5. Method according to claim 3, further comprising the steps of
- acquiring data regarding said one or more microorganisms and data regarding said matrix or said environment,
- performing a risk analysis and assessment in order to determine a risk of disease, contamination or loss by said one or more microorganisms in said matrix or environment, and
- compiling the results of said risk analysis and outputting said results in a report.

6. Method according to any of claims 1 to 5, further comprising the step of recommending an appropriate prevention, remediation or minimization strategy.

7. Method according to any of claims 1 to 6, wherein said report comprises visual codes wherein each code corresponds to a category of risk of disease development, contamination or loss.

8. Method according to any of claims 1 to 7, wherein said report comprises at least three different codes corresponding to three categories of risk of disease development, contamination or loss comprising:
- a first category indicating a no risk of disease development, contamination or loss,
- a second category indicating a low risk of disease development, contamination or loss,
- a third category indicating a high risk of disease development, contamination or loss.

9. Method according to any of claims 1 to 8, wherein said risk analysis and assessment is performed by using a risk analysis and assessment system comprising an adaptive database and analytical tools.

10. Method according to any of claims 1 to 9, wherein said microorganisms comprise organisms selected from the group comprising fungi, actinomycetes, bacteria, mollicutes, protozoa, stramenopiles, nematodes, viruses, viroids, or prions.

11. Method according to any of claims 3 to 10, wherein said targets comprise molecules extracted from a sample, and preferably comprise genomic DNA, genomic RNA or mRNA, whereby the RNA targets are converted into cDNA before being amplified.

12. Method according to any of claims 1 to 11, further comprising the amplification of exogenous and/or endogenous control molecules, preferably exogenous and/or endogenous DNA.

13. Method according to any of claims 1 to 12, wherein amplification is obtained using a set of primer pairs comprising
- at least one consensus primer pair for amplifying target molecules, preferably DNA, from a specific group of microorganisms, and
- at least one primer pair for amplifying exogenous and/or endogenous control molecules, preferably control DNA.

14. Method according to any of claims 1 to 13, wherein the number of amplification cycles prior to hybridization is adjusted to a number of between 25 and 40 cycles, and preferably to a number of between 25 and 35 cycles.

15. Method according to any of claims 1 to 14, wherein said array contains at least three categories of capture molecules comprising:
- a first category of capture molecules being specific to target molecules,
- a second category of capture molecules being specific to (exogenous and/or endogenous) control molecules; and
- a third category of capture molecules comprising labeled molecules for calibration of hybridization signals.

16. Use of an array comprising a solid support having capture molecules bound there on for the detection, identification and quantification of microorganisms or components thereof in a matrix or environment, wherein said array contains at least three categories of capture molecules, as defined in claim 15.

17. Risk analysis report obtainable by a method according to any of claims 1 to 15, wherein said report comprises visual codes whereby each code corresponds to a category of risk of disease development, contamination or loss, and a suitable prevention, remediation or minimization strategy.

18. System for assessing and reporting a risk of disease development, contamination or loss, by one or more microorganisms in a matrix or environment comprising:
- data acquisition means for acquiring data on one or more microorganisms and on said matrix or environment:
- a risk analysis and assessment system for analyzing and assessing the risk of disease development, contamination or loss, by said one or more microorganisms, and
- a risk reporting system for outputting the results of said risk analysis and assessment in a suitable report.

19. Diagnostic kit for the detection, identification and quantification of one or more microorganisms or components thereof in a matrix or environment, comprising:
- suitable means and media for isolating targets, and preferably genomic DNA, genomic RNA, or mRNA from said sample;
- a set of primer pairs as defined in claim 13, and
- an array preferably containing at least three categories of molecules as defined in claim 15 that are spotted on a solid support.


SEQUENCE LISTING

<110>  De Ceuster NV

<120>  Reliable method and system for assessing and reporting the risks of
disease, contamination and losses caused by one or more microorganisms in a
matrix or environment

<130>  TER-001-EP-PRIO1

<160>  17

<170>  PatentIn version 3.1

<210>  1
<211>  21
<212>  DNA
<213>  Artificial Sequence

<220>
<223>  probe
<400>  1
gttgggactc gcgttaattc g                                                21

<210>  2
<211>  23
<212>  DNA
<213>  Artificial Sequence

<220>
<223>  probe
<400>  2
atcaaccctg tgaacatacc taa                                              23

<210>  3
<211>  27
<212>  DNA

<213>  Artificial Sequence

<220>

<223>  probe

<400>  3
ttggagtata gatcagtatt aggtaaa                                          27

<210>  4

<211>  24

<212>  DNA

<213>  Artificial Sequence

<220>

<223>  probe

<400>  4
catcagtctc tttattcata ccaa                                             24

<210>  5

<211>  21

<212>  DNA

<213>  Artificial Sequence

<220>

<223>  probe

<400>  5
aacagagaga ctgatggacc g                                                21

<210>  6

<211>  20

<212>  DNA

<213>  Artificial Sequence

<220>

<223>  probe

<400>  6
gctgcgttct tcatcgatgc                                                  20

<210>  7

<211>  22

<212>  DNA

<213>  Artificial Sequence

<220>

<223>  probe

<400>  7
gtgttttgga tggtggtaag aa                                                22

<210>  8

<211>  20

<212>  DNA

<213>  Artificial Sequence

<220>

<223>  probe

<400>  8
tcctccgctt attgatatgc                                                   20

<210>  9

<211>  21

<212>  DNA

<213>  Artificial Sequence

<220>

<223>  probe

<400>  9
gtccagacag gatcaggatt g                                                 21

<210>  10

<211>  21

<212>  DNA

<213>  Artificial Sequence

<220>

<223>  probe

<400>  10
gtccagacag gatcaggatt g                                                 21

<210>  11

<211>  20

<212>  DNA

<213>  Artificial Sequence

<220>

<223>  primer

<400>  11
gcagcgttct tcatcgatgt                                                    20

<210>  12

<211>  21

<212>  DNA

<213>  Artificial Sequence

<220>

<223>  primer

<400>  12
cgaattaacg cgagtcccaa c                                                  21

<210>  13

<211>  21

<212>  DNA

<213>  Artificial Sequence

<220>

<223>  primer

<400>  13
ggtatgttca cagggttgat g                                                  21

<210>  14

<211>  20

<212>  DNA

<213>  Artificial Sequence

<220>

<223> primer

<400> 14
caatgcaaag tcagcagtgc 20

<210> 15

<211> 19

<212> DNA

<213> Artificial Sequence

<220>

<223> primer

<400> 15
tgtatggaga cgctgcatt 19

<210> 16

<211> 21

<212> DNA

<213> Artificial Sequence

<220>

<223> primer

<400> 16
agtgttatgc ttggttccac t 21

<210> 17

<211> 19

<212> DNA

<213> Artificial Sequence

<220>

<223> primer

<400> 17
cagagagact gatggaccg 19